(19) **Europäisches Patentamt** / **European Patent Office** / **Office européen des brevets**

(11) **EP 4 145 124 B1**

(12) **EUROPEAN PATENT SPECIFICATION**

(45) Date of publication and mention of the grant of the patent:
**10.09.2025   Bulletin 2025/37**

(21) Application number: **22193755.0**

(22) Date of filing: **02.09.2022**

(51) International Patent Classification (IPC):
**G01N 30/46** (2006.01)   **B01D 15/34** (2006.01)
**G01N 30/88** (2006.01)   **G01N 33/15** (2006.01)
**B01D 15/18** (2006.01)   **A61K 39/00** (2006.01)
**G01N 30/74** (2006.01)

(52) Cooperative Patent Classification (CPC):
**G01N 30/461; A61K 39/00; B01D 15/1871;**
**B01D 15/34; G01N 30/88;** G01N 30/74;
G01N 2030/8836; G01N 2030/8872;
G01N 2030/8886

(54) **METHOD FOR MONITORING STABILITY OF POLYSACCHARIDE-PROTEIN CONJUGATE VACCINES**

VERFAHREN ZUR ÜBERWACHUNG DER STABILITÄT VON POLYSACCHARID-PROTEIN-KONJUGAT-IMPFSTOFFEN

PROCÉDÉ DE SURVEILLANCE DE LA STABILITÉ DE VACCINS CONJUGUÉS POLYSACCHARIDE-PROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **04.09.2021   IN 202121040146**

(43) Date of publication of application:
**08.03.2023   Bulletin 2023/10**

(73) Proprietor: **Serum Institute of India Private Limited**
**Pune 411 028**
**MAH (IN)**

(72) Inventors:
- **GAIROLA, SUNIL JAGDISHPRASAD**
  **411028 Pune Maharashtra (IN)**
- **GOEL, SUNIL KUMAR**
  **411028 Pune Maharashtra (IN)**
- **SHARMA, PANKAJKESHAV**
  **411028 Pune Maharashtra (IN)**
- **KALE, SAMEER MANOHAR**
  **411028 Pune Maharashtra (IN)**
- **PENDHARKAR, SUMIT VILAS**
  **411028 Pune Maharashtra (IN)**
- **SHINDE, MADHUKAR PRASAD**
  **411028 Pune Maharashtra (IN)**
- **MANDHAN, AARUSHI**
  **411028 Pune Maharashtra (IN)**

(74) Representative: **Studio Torta S.p.A.**
**Via Viotti, 9**
**10121 Torino (IT)**

(56) References cited:
- **THIEBAUD JEROME ET AL: "Development and validation of high-performance size exclusion chromatography methods to determine molecular size parameters of Haemophilus influenzae type b polysaccharides and conjugates", ANALYTICAL BIOCHEMISTRY, vol. 453, 15 May 2014 (2014-05-15), pages 22 - 28, XP093015494**
- **JUMEL K ET AL: "EVALUATION OF MENINGOCOCCAL C OLIGOSACCHARIDE CONJUGATE VACCINES BY SIZE-EXCLUSION CHROMATOGRAPHY/MULTI-ANGLE LASER LIGHT SCATTERING", BIOTECHNOLOGY AND APPLIED BIOCHEMISTRY, ACADEMIC PRESS, US, vol. 36, no. 3, 1 December 2002 (2002-12-01), pages 219 - 226, XP009058011, ISSN: 0885-4513, DOI: 10.1042/BA20020066**

- **PLUMB J E ET AL: "Molecular size characterization of Haemophilus influenzae type b polysaccharide-protein conjugate vaccines", VACCINE, ELSEVIER, AMSTERDAM, NL, vol. 14, no. 5, 1 April 1996 (1996-04-01), pages 399 - 404, XP004057295, ISSN: 0264-410X, DOI: 10.1016/0264-410X(95)00202-C**
- **VON HUNOLSTEIN C ET AL: "A routine high-performance size-exclusion chromatography to determine molecular size distribution of Haemophilus influenzae type b conjugate vaccines", VACCINE, ELSEVIER, AMSTERDAM, NL, vol. 17, no. 2, 1 January 1999 (1999-01-01), pages 118 - 125, XP004139966, ISSN: 0264-410X, DOI: 10.1016/S0264-410X(98)00166-2**

## Description

### TECHNICAL FIELD:

The present disclosure is related to polysaccharide - carrier protein conjugate vaccines manufacturing and evaluation of stability and quality of conjugate vaccines.

### BACKGROUND:

Vaccines based on capsular polysaccharides stimulate humoral immunity but fail to induce immunologic memory, and thus are ineffective in young infants. First generation vaccines were based on bacterial capsular polysaccharides; yet, most of these antigens are considered T-independent antigens, showing significant gaps in terms of immunogenicity, particularly with respect to the generation of the immune memory. The development of protein-polysaccharide conjugation technology in the 1980s allowed the availability of novel vaccines against Haemophilus influenzae type b (Hib), Streptococcus pneumoniae and different serogroup of Neisseria meningitidis that have demonstrated a very good safety and tolerability, together with the capability of eliciting a strong immunogenicity combined with the demonstration of the anamnestic antibody responses.

For a vaccine to be effective and induce long lasting immunity, induction of T cell memory is very important. Increased immunogenic response of an antigen can be achieved by converting the T-independent antigen to T-dependent antigen. The conjugation of polysaccharide to carrier protein renders the antigenic molecule to undergo the process of antigen presentation and enhance the polysaccharide immunogenicity by eliciting the T-cell dependent response. The proteins used for conjugation to polysaccharides include CRM197, tetanus toxoid, diphtheria toxoid, Neisseria meningitidis outer membrane complex, Haemophilus influenzae protein D, Pneumolysin, etc.

The meningococcal meningitis is caused by Neisseria meningitidis (meningococcal) which is an aerobic Gram-negative encapsulated bacterium. To date, more than 10 serotypes of meningococcal have been characterized by differences in the polysaccharide capsule. The polysaccharides from serotype A, C, Y, W, & X have been conjugated to various carrier proteins to prepare a polysaccharide-protein conjugate vaccine (Menactra®, Menveo®, Nimenrix®, MenQuadFi®), effective against infection by these serotypes.

Similarly, for Streptococcus pneumoniae, more than 90 distinct serotypes have been identified throughout the world (WHO); of which a small number of serotypes accounts for most diseases in infants. Pneumococcal conjugate vaccines containing poly-saccharide from 7+ serotypes i.e. Prevnar® & Synflorix® are already into market. The polysaccharides from S. pneumoniae serotype have been conjugated to various carrier proteins i.e. CRM197, Protein D, TT, DT, etc. to prepare a conjugate vaccine against these serotypes.

Synflorix®, a licensed 10 valent Pneumococcal polysaccharide conjugate vaccine comprises Ps conjugated to three different carrier proteins. Polysaccharide from serotype 1, 4, 5, 6B, 7F, 9V, 14 and 23F are conjugated to protein D (derived from non-type able Haemophilus influenzae) carrier protein; Polysaccharide from serotype 18C is conjugated to tetanus toxoid carrier protein; and polysaccharide from serotype 19F is conjugated to diphtheria toxoid carrier protein. A 11 valent Pneumococcal conjugate vaccine comprising Ps conjugated to at least two different carrier proteins has also been reported (Ref: Rose-Marie Olandera et al 2002; Booster response to the tetanus and diphtheria toxoid carriers of 11-valent pneumococcal conjugate vaccine in adults and toddlers; Vaccine 20; 336-341) wherein polysaccharide is selected from 1, 3, 4, 5, 6B, 7F, 9V, 14, 18C, 19F and 23F and carrier proteins are Diphtheria toxoid and tetanus toxoid. Further, Serum Institute of India's Penumosil® is a licensed 10 valent Pneumococcal polysaccharide conjugate vaccine that comprises of polysaccharide from serotype 1, 5, 6A, 6B, 7F, 9V, 14 19A, 19F and 23F individually conjugated to CRM197.

Also, a pentavalent Meningococcal conjugate vaccine comprising of Ps conjugated to two different carrier proteins is being developed, wherein polysaccharide from serotype A and X are individually conjugated to tetanus toxoid and polysaccharide from serotype C, Y, W are individually conjugated to CRM197.

Polysaccharide-protein conjugates are relatively more stable compared to live attenuated bacterial vaccines but the polysaccharide (PS) chains of such conjugates tend to degrade by hydrolysis. The potency of these vaccines depends on effective conjugation of the saccharide to a protein carrier and preservation of the integrity of the conjugate molecule during shelf life. Thereby, factors adversely affecting conjugate stability may diminish vaccine potency by reducing the amount, accessibility, and solubility of the conjugated saccharide and carrier protein epitopes, limiting their protective efficacy. High temperature is known to cause physical instability, such as altered tertiary structure and protein aggregation, as well as chemical instability, such as dissociation of polysaccharides from the protein carrier, in this kind of vaccine. These changes can be assessed under thermal stress, the most widespread adverse condition used in the industry.

Polysaccharide protein conjugate vaccine composition are known to be associated with contaminants such as un-reacted polysaccharide (free polysaccharide), un-reacted carrier protein (free protein), low molecular weight conjugates, and other chemicals used for affecting conjugation such as linkers, coupling agents etc. are highly undesirable in a product which is intended for use as a vaccine.

Furthermore, studies under stress conditions may be useful in determining whether short-term accidental exposure to undesired conditions, such as improper transportation, may compromise product quality. Therefore, effective methods are essential for monitoring quality and detecting changes that may potentially affect the efficacy of these vaccines. Establishing stability indicators for conjugate vaccine molecules is a key step to ensure their quality. Physicochemical analysis methods are widely used in pre- and post-licensing quality control of these vaccines to ensure compliance with manufacturing specifications and Lot-to-lot (or batch-to-batch) product consistency. Understanding stability of the molecule helps in the selection of a proper formulation and package as well as providing proper storage conditions and shelf life, which is essential from regulatory/licensure perspective.

Thus, the Food and Drug Administration and International Conference on harmonization guidelines state the requirement of stability testing data to understand how the quality of a drug substance (i.e., bulk material) and drug product changes with time under the influence of various environmental factors. There is no single stability-indicating assay or parameter that provides the stability characteristics of a biotechnological/biological product. Consequently, a stability profile should be proposed ensuring detection of changes in product identity, purity, and potency.

Adequate stability studies form an essential part of the vaccine development studies, they should be designed to help in setting the shelf-life of the vaccines. WHO has developed further guidance on the stability studies of vaccines and general principles should be followed. Stability indicate separately all relevant details including changes in the proportion of free saccharide, molecular size distribution, pH, residual moisture and immunogenicity in accelerated degradation tests, and after storage for the maximum period claimed for the product at the recommended temperature.

The stability of intermediate products such as the purified polysaccharide and the bulk conjugate before mixing into the final bulk must be demonstrated. The stability of the vaccine in its final form, with final containers and at the recommended storage temperatures should be demonstrated to the satisfaction of the national regulatory authorities for at least three lots of final product manufactured from different independent bulk conjugates. In addition, a real-time stability study should be conducted on at least one final container lot produced per year.

The saccharide component of conjugate vaccines may be subject to gradual hydrolysis at a rate which may vary depending upon the type of conjugate, the type of formulation or adjuvant, the types of excipient and conditions of storage. The hydrolysis may result in reduced molecular size of the saccharide component, a reduction in the amount of the saccharide bound to the carrier protein and/or in a reduced molecular size of the conjugate. The structural stability of the saccharide chains and of the protein carrier varies between different conjugate vaccines as found for group C meningococcal conjugate vaccines.

Tests should be conducted before licensing to determine the extent to which the stability of the product has been maintained throughout the proposed validity period. The free saccharide content as a percentage of the total saccharide should be determined. The vaccine should meet the recommendations for final product up to the expiry date. Molecular sizing of the final product and estimation of free saccharide may be carried out to ensure the integrity of the conjugate.

Free or unconjugated polysaccharides that are not bound to the carrier proteins also exist in a conjugate vaccine due to incomplete conjugation reaction. Since these free polysaccharides are identical to the polysaccharide only vaccines, they may act as contaminants by interfering with the immunological responses induced by polysaccharide-protein conjugates. Thus, it is difficult to achieve increased immunogenicity when the amount of free polysaccharide exceeds that of the conjugated polysaccharide. Therefore, the low level of free polysaccharide content is an important control to preserve vaccine immunogenicity.

Commonly used methods for free polysaccharide separation in conjugate vaccines, are ammonium sulphate/ethanol/CTAB/deoxycholate conjugate precipitation, capillary electrophoresis, ultrafiltration, reverse phase-solid phase extraction separation, ion exchange chromatography, hydrophobic interaction chromatography and affinity chromatography.

Suker et al. 2004 mentions that the development of control tests for MenC conjugate vaccines has in many respects been based on the recommendations for Hib conjugate vaccines and places emphasis on determining the integrity of the conjugate by physicochemical methods such as nuclear magnetic resonance (NMR) spectroscopy, size exclusion chromatography (SEC) and high performance anionic exchange chromatography with pulsed amperometric detection (HPAEC-PAD). Immunogenicity assays have been valuable in conjugate vaccine development but are not necessary for routine batch-release, where vaccine consistency has been demonstrated by other means.

Assays of molecular size by SEC can be sensitive to batch-to-batch changes, although the accuracy of molecular-weight values determined using protein or saccharide standards is poor. The addition of light-scattering detectors to the chromatographic system gives much more accurate molecular-mass values than the values obtained from the use of concentration detectors, such as UV or refractive index alone. The key control test for glycoconjugate vaccines is free saccharide determination, which is considered to be the critical assay to evaluate conjugate stability. First, the free saccharide is separated from conjugated saccharide, for example by ultrafiltration, interaction with hydrophobic beads, or detergent precipitation. The quantity of saccharide is then measured by HPAEC-PAD in both the separated free saccharide portion and the conjugate sample before separation, in order to determine the percentage of free saccharide in the original vaccine sample.

Final vaccine composition (Drug product) comprises antigens and excipient/stabilizer and preservative. During quality control assays, it is difficult to analyze antigens in presence of these components which may cause interference during analysis. In addition to the problems related to desorption or dissolution of adjuvant, high concentrations of salts or aluminum complexes may remain on chromatographic equipment and could potentially interfere with assay procedures relying on spectrophotometric measurement. This has driven refinements to the batch-release testing schedule such that molecular size is measured at the bulk conjugate rather than final-fill stage comprising multiple antigens of similar molecular weight that gets mixed up to make a formulation. This strategy is also used in the evaluation of multivalent pneumococcal conjugates vaccines and will be considered for meningococcal conjugate combinations (e.g., A/C or ACW-135Y). However, as formulations and combinations become more complex, it will be important for manufacturers to rule out that significant changes in immunogenicity and the physicochemical profile occur as a result of component interactions, formulation or filling.

According to Annex_2_WHO_TRS_962 - The molecular size of the saccharide-protein conjugate is an important parameter in establishing consistency of production and in studying stability during storage. The relative molecular size of the saccharide-protein conjugate should be determined for each bulk conjugate, using a gel matrix appropriate to the size of the conjugate. The method should be validated with an emphasis on specificity to distinguish the saccharide-protein conjugate from other components that may be present, e.g. unbound protein or free saccharide for monovalent glycoconjugate vaccine.

Typically, the size of the saccharide-protein conjugate may be examined by methods such as gel filtration on Sepharose CL-4B, or by HPSEC on an appropriate column. Since the saccharide to protein ratio is an average value, characterization of this ratio over the size distribution (e.g. by dual monitoring of the column eluent) can be used to provide further proof of manufacturing consistency.

Physicochemical methods to measure molecular size and amount of free saccharide are used as the main control tests and indicators of product conformity for monovalent polysaccharide protein conjugate vaccines. It is anticipated that monovalent polysaccharide protein conjugate vaccines will in future be replaced by multi-component products which will require more extensive testing to ensure that vaccine quality is not compromised.

K. Jumel et al. 2002 discloses Men C - CRM197 conjugate molecular mass determination by SEC-MALLS (Mol size determined by GPC, Avg Mol wt Men C conjugate 74.9 kDa $\pm$ 5 kDa). LEMERCINIER ET AL. 1997 discloses mol wt determination of Hib conjugate and Men CCRM197 conjugate by FPLC. M.M. Ho et al. 2001 discloses FPLC-SEC and SEC-MALLS/RI to determine size of Men C - CRM197 conjugate and molecular mass.

Multivalent polysaccharide vaccines contain many immunogenic components, each of which must be characterized separately. At present, no suitable assay for quality control of these multi-component mixtures is available wherein the final quality control technique is traceable to reliable analytical methods such as SEC-MALS.

Nimenrix EMEA (Procedure No. EMEA/H/C/002226) 2011 discloses molecular size distribution (MSD) for the commercial batches of MenACWY-TT at the 9 months, 18 months & acknowledges that free PS, MSD analysis, both are indicative of degradation and aggregation. It also mentions that integrity of the conjugates with respect to degradation, the MSD assay might be one of the more appropriate methods applied.

Aggregate formation or aggregate molecules are observed during manufacturing or during storage period of vaccine at various conditions. Aggregate molecules contribute to high molecular weight (HMW) and low molecular weight (LMW) molecules. Conjugated carrier proteins also appeared to form oligomers or 'aggregates' at elevated temperatures. Polysaccharide-polysaccharide aggregates are also encountered. Free polysaccharide analysis method as the in-process and stability evaluation parameter mostly provides information about the free polysaccharide content in the vaccine sample and does not give information related to such aggregate formation/molecules present in the vaccine sample. Applicant has found that aforementioned aggregates have an adverse impact on both the stability and immunogenicity of polysaccharide-protein conjugate vaccine. There is an unmet need for monitoring conjugate vaccines across manufacturing and storage for profiling of such aggregates in addition to free polysaccharide measurement.

**OBJECT OF INVENTION:**

The scope of the invention is defined by the appended claims.

Some of the objects of the present disclosure, which at least one embodiment herein satisfies, are as follows:

- An object of the present disclosure is to ameliorate one or more problems of the prior art or to at least provide a useful alternative.
- An object of the present disclosure is to provide a stable vaccine composition/formulation suitable for the prevention and treatment of more than one disease state and meets the criterion for the optimal seroprotection.
- An object of the present disclosure is to provide an assay indicating stability parameter like free PS estimation method during stability evaluation of formulation which provides Molecular size distribution and Molar mass distribution data of a vaccine composition.

- An object of the present disclosure is to provide an SEC-MALS based assay indicating stability parameter like free PS estimation method during stability evaluation of formulation which provides Molecular size distribution and Molar mass distribution data of a vaccine composition.
- An object of present disclosure to provide an assay for stability testing of monovalent polysaccharide-carrier protein conjugate vaccine.
- An object of present disclosure to provide an assay for stability testing of multivalent polysaccharide-carrier protein conjugate vaccine.
- An object of present disclosure to provide an assay for determination of vaccine quality and immunogenicity of polysaccharide-carrier protein conjugate vaccine.
- An object of present disclosure to provide a polysaccharide-carrier protein conjugates vaccine composition with a defined pattern of molar mass distribution and molecular size distribution and having improved stability and immunogenicity.
- An object of present disclosure to provide a multivalent polysaccharide-carrier protein conjugate vaccine composition with improved stability and immunogenicity.
- An object of present disclosure to provide a polysaccharide-carrier protein conjugate vaccine composition with two or more carrier proteins.
- Applicant has found a method for profiling of conjugates based on molecular size and/or molar mass in a multivalent N. Meningitidis conjugate vaccine (ACWYX) drug product using SEC-HPLC-MALS; showing correlation between Molecular Size Distribution (SEC-MALS) and Stability [comprising profiling in terms of Average Molecular Weight/-AMW, High Molecular Weight/ HMW, Low Molecular Weight/LMW molecules] which contribute to determining lot-to-lot consistency, stability of multivalent drug product contributing to integrity, degradation and aggregation profile of multivalent drug product and its ingredients.
- Particularly applicant has found that such molecular size distribution (measurement & profiling of a multivalent in terms of AMW, HMW, LMW) is an effective alternative method (in addition to free polysaccharide measurement) to assess stability of multivalent conjugate mix ACWYX (drug product).
- Applicant has found a novel approach/assay for measuring aggregation profile & molecular size distribution of a multivalent drug product mix and is done in the presence of two carrier proteins, free polysaccharides and excipient, without any interference of said components.
- Applicant's assay utilizes set of three columns [Shodex 807 + Shodex 806 + TSK gel G5000 PwxL / TSK gel G6000 PwxL + with guard column] in a series, using Phosphate buffer as mobile phase along with optimized flow rate, time, temperature and detectors [UV; RI; & MALS].

## SUMMARY OF INVENTION:

Novel Multicomponent products (multivalent polysaccharide protein conjugate vaccines) will require more extensive testing to ensure that vaccine quality is not compromised. In Multicomponent products (multivalent polysaccharide protein conjugate vaccines), free polysaccharide and free protein are individually/separately considered as stability indicating parameters for which individual tests are to be carried out. At present, no known method exists for measuring aggregation profile & molecular size distribution of conjugates in a multivalent drug product to support the lot to lot consistency and to an extent confirm the stability & integrity of the multivalent polysaccharide protein conjugate vaccine formulation (ACWYX comprising of N. meningitidis serogroup X in particular). Applicant has found a method comprising use of SEC-HPLC-MALS for profiling of conjugates based on molecular size and/or molar mass in a multivalent N. Meningitidis conjugate vaccine (ACWYX) drug product in terms of Average Molecular Weight/AMW, High Molecular Weight/HMW, Low Molecular Weight/LMW molecules showing correlation between Molecular Size Distribution (SEC-HPLC-MALS) and Stability which ultimately contributes to determining integrity, degradation and aggregation profile of multivalent drug product and its ingredients.

Particularly applicant has found that such molecular size distribution (measurement & profiling of a multivalent in terms of AMW, HMW, LMW) is an effective alternative method (in addition to free polysaccharide and free protein measurement) to assess stability of multivalent conjugate mix ACWYX (drug product). The novel assay for measuring aggregation profile & molecular size distribution of a multivalent drug product mix is done in the presence of two carrier proteins, free polysaccharides and excipient, without any interference of said components. Applicant's assay utilizes set of three columns [Shodex 807/Shodex 806 + Shodex 806/Mixed Bead columns + TSK gel 6000/5000 PwxL + with guard column] in a series, using Phosphate buffer as mobile phase along with optimized flow rate, time, temperature and detectors [UV; RI; & MALS].

## BREIF DESCRIPTION OF DRAWING:

The present disclosure will now be described with the help of the accompanying drawing, in which:

Figure 1: Illustrates the stability studies of MenFive (Lyophilized) vaccine 1 Dose at 2-8°C (initial), 25°C (1 month), 40°C (1 month), 60°C (1 month)

Figure 2: Illustrates the stability studies of MenFive (Lyophilized) vaccine 5 Dose at 2-8°C (initial), 25°C (1 month), 40°C (1 month), 60°C (1 month)

Figure 3: Illustrates the comparison of stability studies at 2-8°C (initial) between MenFive Lyophilized vaccine 1 Dose and MenFive Liquid vaccine 1 Dose

Figure 4: Illustrates the comparison of stability studies at 25°C (1 month) between MenFive Lyophilized vaccine 1 Dose and MenFive Liquid vaccine 1 Dose

Figure 5: Illustrates the comparison of stability studies at 40°C (1 month) between MenFive Lyophilized vaccine 1 Dose and MenFive Liquid vaccine 1 Dose

Figure 6: Illustrates the comparison of stability studies at 60°C (1 month) between MenFive Lyophilized vaccine 1 Dose and MenFive Liquid vaccine 1 Dose

Figure 7: Illustrates the comparison of stability studies at 2-8°C (initial) between MenFive Lyophilized vaccine 5 Dose and MenFive Liquid vaccine 5 Dose

Figure 8: Illustrates the comparison of stability studies at 25°C (initial) between MenFive Lyophilized vaccine 5 Dose and MenFive Liquid vaccine 5 Dose

Figure 9: Illustrates the comparison of stability studies at 40°C (initial) between MenFive Lyophilized vaccine 5 Dose and MenFive Liquid vaccine 5 Dose

Figure 10: Illustrates the comparison of stability studies at 60°C (initial) between MenFive Lyophilized vaccine 5 Dose and MenFive Liquid vaccine 5 Dose

Figure 11: Illustrates HPLC-SEC chromatogram of 1 Dose Lyophilized vaccine at 2-8, 25, 40 and 60°C (UV Detector)

Figure 12: Illustrates HPLC-SEC chromatogram of 1 Dose Lyophilized vaccine at 2-8, 25, 40 and 60°C (RI Detector)

Figure 13: Illustrates HPLC-SEC chromatogram of 1 Dose Lyophilized vaccine at 2-8, 25, 40 and 60°C (LS Detector)

Figure 14: Illustrates HPLC-SEC chromatogram of 1 Dose Liquid vaccine at 2-8, 25, 40 and 60°C (UV Detector)

Figure 15: Illustrates HPLC-SEC chromatogram of 1 Dose Liquid vaccine at 2-8, 25, 40 and 60°C (RI Detector)

Figure 16: Illustrates HPLC-SEC chromatogram of 1 Dose Liquid vaccine at 2-8, 25, 40 and 60°C (LS Detector)

Figure 17: Illustrates HPLC-SEC chromatogram of 5 Dose Lyophilized vaccine at 2-8, 25, 40 and 60°C (UV Detector)

Figure 18: Illustrates HPLC-SEC chromatogram of 5 Dose Lyophilized vaccine at 2-8, 25, 40 and 60°C (RI Detector)

Figure 19: Illustrates HPLC-SEC chromatogram of 5 Dose Lyophilized vaccine at 2-8, 25, 40 and 60°C (LS Detector)

Figure 20: Illustrates HPLC-SEC chromatogram of 5 Dose Liquid vaccine at 2-8, 25, 40 and 60°C (UV Detector)

Figure 21: Illustrates HPLC-SEC chromatogram of 5 Dose Liquid vaccine at 2-8, 25, 40 and 60°C (RI Detector)

Figure 22: Illustrates HPLC-SEC chromatogram of 5 Dose Liquid vaccine at 2-8, 25, 40 and 60°C (LS Detector)

## DETAILED DESCRIPTION:

Although the present disclosure may be susceptible to different embodiments, certain embodiments are shown in the drawing and following detailed discussion, with the understanding that the present disclosure can be considered an exemplification of the principles of the disclosure and is not intended to limit the scope of disclosure to that which is illustrated and disclosed in this description.

Embodiments are provided so as to thoroughly and fully convey the scope of the present disclosure to the person skilled in the art. Numerous details are set forth, relating to specific components, and processes, to provide a complete understanding of embodiments of the present disclosure. It will be apparent to the person skilled in the art that the details provided in the embodiments should not be construed to limit the scope of the present disclosure. In some embodiments, well-known composition, well-known processes, and well-known techniques are not described in detail.

The terminology used, in the present disclosure, is only for the purpose of explaining a particular embodiment and such terminology shall not be considered to limit the scope of the present disclosure. As used in the present disclosure, the forms "a," "an," and "the" may be intended to include the plural forms as well, unless the context clearly suggests otherwise.

The terms "comprises," "comprising," "including," and "having," are open ended transitional phrases and therefore specify the presence of stated features, integers, steps, operations, elements, modules, units and/or components, but do not forbid the presence or addition of one or more other features, integers, steps, operations, elements, components, and/or groups thereof. The particular order of steps disclosed in the process of the present disclosure is not to be construed as necessarily requiring their performance as described or illustrated. It is also to be understood that additional or alternative steps may be employed.

The terms first, second, third, etc., should not be construed to limit the scope of the present disclosure as the aforementioned terms may be only used to distinguish one element, component, region, layer or section from another component, region, layer or section. Terms such as first, second, third etc., when used herein do not imply a specific sequence or order unless clearly suggested by the present disclosure. The present disclosure provides an immunogenic composition and a process for preparing the same.

As used herein the terms "Freeze-drying" or "lyophilize" or "lyophilisation" involves lyophilization and refers to the process by which a suspension is frozen, after which the water is removed by sublimation at low pressure.

An embodiment of the disclosure provides an evaluation of stability and quality of polysaccharide-carrier protein conjugate vaccine composition.

An embodiment of the present disclosure provides designing and development of an assay for determination of stability indicating parameter. In an aspect of the present embodiment, the disclosure provides determination of molecular size and/or weight in polysaccharide-carrier protein conjugate vaccine composition. In another aspect of the present embodiment disclosure provides to understand lot to lot consistency of vaccine composition by determining and analysing information on distribution of various molecules (conjugates) in terms of presence of high molecular weight (HMW) and low molecular weight (LMW) in the vaccine composition. In another aspect of the said embodiment, it is provided that molar mass distribution of the vaccine composition may provide an information on the confirmation of the molecules through its presentation as HMW, LMW, aggregates and the presentation of majority of molecules of particular molecular weight and its contribution in the vaccine sample. In another aspect of the present embodiment, the distribution pattern of the molecules present in a vaccine composition may help to elucidate the characteristics/attributes of different dose presentations and formulations. The present disclosure further provides elucidating characteristics/attributes in terms of molecular weight/molar mass and molecular size of the various liquid and lyophilized vaccine compositions.

An embodiment of the present disclosure, it is provided that the vaccine composition stored at various temperature ranges may be analyzed to study, analyze and correlate the molecular size distribution & molar mass distribution with stability and immunogenicity of the vaccine composition. It is further provided that the vaccine composition may be stored at temperature between -80 to 55 Degree Celcius.

An embodiment of the present disclosure provides an assay for elucidation of characteristics/attributes in a vaccine composition including molecular size pattern, molecular size distribution pattern, average molecular weight (AMW), high molecular weight (HMW) and low molecular weight (LMW); and the majority molecules present in a vaccine composition.

It is understood that low molecular weight (LMW) are molecules/components having molecular weight in range between 200 & 2000; average molecular weight (AMW) are molecules/components having molecular weight in range between 2000 & 11000; high molecular weight (HMW) are molecules/components having molecular weight in range between 10000 & 50000 or 13000 & 50000.

An embodiment of the present disclosure provides an assay for elucidation of characteristics / attributes in a vaccine composition wherein the behaviour (characteristics / attributes) of individual conjugates in a multivalent polysaccharide-carrier protein conjugate (and/or multivalent drug product) is not possible. In an aspect of the said embodiment, it is further provided to provide information and to assess / elucidate lot to lot consistency and to generate characterization data to understand the process dynamics of stability indicating parameters in presence of excipient, including presence of molecular size pattern, molecular size distribution pattern, average molecular weight (AMW), high molecular weight (HMW) and low molecular weight (LMW); and the majority molecules present in a vaccine composition.

Size-exclusion chromatography (SEC) is a separation technique based on the molecular size of the components. Separation is achieved by the differential exclusion from the pores of the packing material of the sample molecules as they pass through a bed of porous particles. Size exclusion chromatography (SEC) is preferably used as one of HPLC separation modes, wherein column used is filled with material/resin containing pores of suitable sizes. When dissolved molecules of various sizes flow into the column, smaller dissolved molecules flow more slowly through the column because they penetrate deep into the pores, whereas large dissolved molecules flow quickly through the column because they do not enter the pores. Consequently, larger molecules elute from the column sooner and smaller molecules later, which effectively sorts the molecules by size. This is the separation principle of size exclusion chromatography.

In an embodiment of the present disclosure, vaccine composition is subjected to size-exclusion chromatography (SEC). Size-exclusion chromatography (SEC) used may be selected from gel permeation chromatography (GPC) and/or gel filtration chromatography (GFC). Gel permeation chromatography (GPC), uses a hydrophobic column packing material and a nonaqueous mobile phase (organic solvent) to measure the molecular weight distribution of synthetic polymers, whereas the gel filtration chromatography (GFC) uses a hydrophilic packing material and an aqueous mobile phase to separate, fractionate, or measure the molecular weight distribution of molecules soluble in water, such as polysaccharides and proteins. In an aspect of the said embodiment, it is provided that large molecular size molecules in a vaccine composition elutes out first from the column followed by small molecular size molecule (like unconjugated carrier protein); molecules are eluted out at different retention time on the basis of their size, molecular weight and shows clear separation which is captured and tracked by three different detectors i.e. UV, RI and MALS (Multi angle light scattering). In an aspect of the said embodiment, it is provided that selection of column, run conditions, method/program, mobile phase and chromatogram integration may be optimized.

In an embodiment of the present disclosure, it is provided to study, analyze and correlate the molecular size distribution and absolute molecular weights of the distributed components of a vaccine composition. In an aspect of the said embodiment, it is further provided to assess / elucidate and characterize the vaccine composition based on molecular size distribution with respect to absolute molecular weight and percent molar mass distribution.

It is provided to further study, analyze and correlate the molecular size distribution & molar mass distribution with immunogenicity of the vaccine composition. In an embodiment, it is provided to further study, analyze and correlate range (%) of average molecular weight (AMW) with stability, immunogenicity and potency/efficacy of the vaccine composition. According to the invention, it is provided to correlate range (%) of High molecular weight (HMW) and Low molecular weight (LMW) with stability, immunogenicity and potency/efficacy of the vaccine composition, or to correlate ratio of average molecular weight (AMW), high molecular weight (HMW) and Low molecular weight (LMW) with stability, immunogenicity and potency/efficacy of the vaccine composition. In a further aspect of the present embodiment, it is further provided to study the interference of excipient in a vaccine composition during analysis of vaccine composition.

In an embodiment of the present disclosure, it is provided to design and optimize a process for size exclusion chromatography (SEC) maintaining specificity and precision. It is understood that the SEC process requires optimization depending on the type of sample to be analyzed. It is understood that the SEC process should be optimized in a manner that there is no interference of the sample components with the column material/resin. It is further understood that the integrity of the sample should remain intact thereby facilitating further analysis of the sample. In other words, process designed should be non-interfering with the sample and should avoid harsh conditions (like high pH buffer, voltage, temperature) for analysis practically maintaining the integrity of molecules/components of sample as it is to provide true presentation of the components/conjugate behaviour in a vaccine composition.

In one of the aspects of the said embodiment, it is provided to use Gel Chromatography for separation of components/molecules present in a vaccine composition. It is understood that Gel chromatography used may be selected from gel filtration chromatography and gel permeation chromatography. Gel permeation chromatography (GPC), which uses a hydrophobic column packing material and a non-aqueous mobile phase (organic solvent) to measure the molecular weight distribution of synthetic polymers. The other is gel filtration chromatography (GFC), which uses a hydrophilic packing material and an aqueous mobile phase to separate, fractionate, or measure the molecular weight distribution of molecules soluble in water, such as polysaccharides and proteins. It is understood that in gel chromatography, large molecular size components/molecule in a vaccine elutes out first from the column followed by small molecular size molecule (Free protein i.e. TT). The molecules are eluted out at different retention time on the basis of their size, molecular weight and show clear separation which is captured and tracked by three different detectors i.e. UV, RI and MALS (Multi angle light scattering). It is further provided that the columns for gel chromatography may be selected on the basis of separation with defined exclusion limits which can resolve very high molecular weight molecules present in a vaccine composition. It is further provided that selection of column is based on consideration that no structural dependency of the molecule is interfering with the final outcome of the process, and lot-to-lot variations in vaccine composition are suitably catered. It is further provided that the process is independent of the inactive components/excipient present in a vaccine composition.

In a multivalent vaccine, the identity of sample remains no more individual from structural and analytical point of view and act as a single component, therefore the individual conjugate containing two carrier protein cannot be discriminated. The assay or method provides the behaviour of total molecules as a whole present in the sample when get mixed to support the lot to lot consistency and to an extent the stability of the mixed formulation or composition.

The invention provides a method for determining the stability of a polysaccharide-protein conjugate vaccine, said method comprising the following steps:

a) subjecting the said polysaccharide-protein conjugate vaccine to a high performance size exclusion chromatography (HPLC-SEC) using a mobile phase and set of three chromatography columns in series to obtain an eluate;
b) passing the said eluate through detectors equipped with the chromatography columns and evaluating the eluate to obtain distribution of the molecules with respect to the molecular weight; and
c) analysing the molecular weight to obtain molecular size and/or molar mass profile of the polysaccharide-protein conjugate vaccine based on the percentage of high molecular weight (HMW), average molecular weight (AMW) and low molecular weight (LMW) molecules.

In an embodiment a pre-treatment step is carried out before step a).

In an embodiment the pre-treatment step comprises of reconstitution of polysaccharide-protein conjugate vaccine using a buffer.

In an embodiment the buffer used for reconstitution of polysaccharide-protein conjugate vaccine in pre-treatment step is selected from phosphate buffer saline, Tris, MES, HEPES, citrate and combinations thereof.

In embodiment the buffer used for reconstitution of polysaccharide-protein conjugate vaccine in pre-treatment step is Tris buffer.

In an embodiment the set of three chromatography columns in series is connected with a guard column.

In an embodiment the arrangement of columns is guard column followed by first column, first column followed by second column and second column followed by third column.

In an embodiment it is provided that the resolution of samples in multivalent (mixed composition) which are likely to be of high molecular weight and obviously complex was important to obtain appropriate molar mass data which is possible by introducing the suitable column with high resolution or exclusion column capabilities.

In a preferred embodiment, considering the range of molar mass and molecular size of the components in a vaccine composition, it is provided to use a combination of 3 columns connected in a series to form a 90 cm long column along with a guard column to ensure that the complete range (HMW to LMW) of molecules are captured for analysis. In a preferred aspect, polymethacrylate based resin columns (Shodex 807, Shodex 806, Shodex mix bead columns SB806M), and polymethacrylate based resin (TSK gel 5000/6000 PwxL) along with guard column in series are provided to be used.

In another embodiment connection of chromatography columns in the series is required to separate out the novel combination of five serogroups with two different carrier proteins. This strategy of connecting the multiple column is formulated considering and selecting the broad exclusion limit which support the separation of vaccine molecules. If columns are not connected in series and are used individually then it is not able to achieve the created dynamic exclusion limit, ultimately affecting the overall analysis or separation which is based on the variable size of molecules present in the vaccine sample. Guard column is not used for the separation purpose but is used to remove particulate contaminants from the sample if present and helps in prolonging the life of the main columns. The usage of guard column is the indication of good chromatographic practices.

In an aspect of the said embodiment the guard column comprises polymer-based packed column for e.g. polyhydroxymethacrylate, etc based material having particle size 9 - 14 $\mu$m.

In an aspect of the said embodiment the first and second chromatography columns comprises polyhydroxymethacrylate based material and third chromatography column is selected from hydroxylated polymethacrylate based material and hydrophilic vinyl polymer based material.

In an aspect of the said embodiment the particle size of first column is 34 - 36 $\mu$m, particle size of second column is 12 - 14 $\mu$m and particle size of third column is 9 - 14 $\mu$m.

In an aspect of the said embodiment the length of the set of three chromatography columns in series ranges from 85 - 95 cm.

In an aspect of the said embodiment the guard column and chromatography columns in series are connected using a connector.

In an aspect of the said embodiment the connector used for connecting guard column and chromatography columns in series is a ferrule.

In another aspect of the said embodiment, it is provided that the eluate is further subjected to analysis using detectors including but not limited to UV (280 nm), Refractive Index (RI), Multiangle light scattering (MALS) or a combination thereof. The detectors used may provide details related to concentration of proteins, presence of aggregates, concentration of polysaccharides and molecular weight, size and mass of the molecules present in a vaccine composition.

In an aspect of the said embodiment the detectors in step b) is selected from UV detector, refractive index (RI) detector and multiangle light scattering (MALS) detector and combinations thereof.

In an aspect of the said embodiment the UV detector was used to track the molecules present in the vaccine having the absorbance at 280 nm. The UV detector was used to track the protein molecules present in the vaccine having the absorbance at 280 nm.

In an aspect of the said embodiment the refractive index (RI) was used to measure the refractive index of the molecules present in the vaccine. The refractive index (RI) was used to measure the refractive index of the polysaccharides present in the vaccine.

In an aspect of the said embodiment the multiangle light scattering (MALS) detector was used to determine the molar mass and molecular weight of the molecules present in the vaccine.

In another aspect of the present embodiment, it is provided to use a mobile phase with non-interference with the components in a vaccine sample; preferably mobile phase used is Phosphate buffer with 200 mM NaCl. It is understood that other suitable buffers may be used as mobile phase.

In an aspect of the said embodiment the mobile phase is a buffer selected from phosphate buffer saline, Tris, MES, HEPES, citrate and combinations thereof.

In an aspect of the said embodiment the mobile phase does not interfere with the vaccine.

In an aspect of the said embodiment the buffer is phosphate buffer saline having 7.2 to 7.5, preferably pH 7.4.

In an aspect of the said embodiment the phosphate buffer saline comprises of sodium chloride in the range of 20 to 40 g, preferably 23.38 g.

In another aspect of the said embodiment, it is provided to use a flow rate lower than the usual ensuring the quality of the chromatography is good and the components in a vaccine composition should get sufficient time to interact with the stationary phase, preferably flow rate is set at 0.1 - 1 ml per minute.

In an embodiment the high performance size exclusion chromatography (HPLC-SEC) in step a) comprises a flow rate ranging from 0.1 to 1 ml per minute, preferably 0.30 to 0.80 ml per minute.

In another aspect of the said embodiment, it is provided to set time parameter in the range of 50 - 300 minutes per injection thereby ensuring the complete separation and recovery of the components in a vaccine composition, and column temperature in the range of 20-40 Degree Celsius thereby ensuring no exceptional temperature is set for analysis making the test procedure simple and temperature independent.

In an embodiment the high performance size exclusion chromatography (HPLC-SEC) in step a) comprises a column temperature ranging from 25°C to 35°C.

In an aspect of the said embodiment the high performance size exclusion chromatography (HPLC-SEC) comprises a column temperature ranging from 25°C to 35°C to ensure that no exceptional temperature is set for analysis and the test procedure was made simple and temperature independent.

In an embodiment the high performance size exclusion chromatography (HPLC-SEC) in step a) comprises injection run time ranging from 50 - 160 min to ensure the complete separation and recovery of the sample.

In an embodiment the high performance size exclusion chromatography (HPLC-SEC) in step a) comprises injection run time ranging from 60 - 70 min.

According to the invention, for lyophilized vaccine low molecular weight (LMW) are molecules/components having molecular weight in range between 200 & 2000; average molecular weight (AMW) are molecules/components having molecular weight in range between 2000 & 11000; high molecular weight (HMW) are molecules/components having molecular weight in range between 13000 & 19000.

According to the invention, for liquid vaccine low molecular weight (LMW) are molecules/components having molecular weight in range between 200 & 1000; average molecular weight (AMW) are molecules/components having molecular weight in range between 1000 & 10000; high molecular weight (HMW) are molecules/components having molecular weight in range between 10000 & 19000.

In an embodiment of the present disclosure, optimized assay is provided to provide characteristics/attributes of each component (AMW, LMW, HMW) present in a vaccine composition wherein no other methods of analysis of components is feasible.

To arrive at the distribution cut the following mathematical calculation is considered

$$K_D \ (0.5) = V_e\text{-}V_o \ / \ V_t\text{-}V_0$$

$$V_e = 0.5 \ X \ (V_t\text{-}V_o) + V_o$$

Where,

$K_D$ = Distribution coefficient.
$V_e$ = Elution volume
$V_o$ = Void volume
$V_t$ = total bed volume
An elution volume ($V_e$) is where the peak is cut to get percent (%) molecular size distribution.
For HMW: $V_e$ is 0.1 $K_D$
For LMW: $V_e$ is 0.5 $K_D$
For AMW: Peak start to peak end is considered.

According to the invention, the high molecular weight is in the range from 13000 kDa to 19000 kDa, average molecular weight is in the range of 2000 kDa to 11000 kDa and low molecular weight is in the range of 200 kDa to 2000 kDa for lyophilized polysaccharide-protein conjugate vaccine.

According to the invention, the high molecular weight is in the range from 10000 Kda to 19000 Kda, average molecular weight is in the range of 1000 Kda to 10000 Kda and low molecular weight is in the range of 200 to 1000 Kda for liquid polysaccharide-protein conjugate vaccine.

An embodiment of the disclosure provides a polysaccharide-carrier protein conjugate vaccine composition.

In an aspect of the present embodiment vaccine composition may comprise of combination of different conjugates having multiple polysaccharides and carrier proteins.

An embodiment of present disclosure provides a polysaccharide-carrier protein conjugate vaccine composition/formulation suitable for the prevention and treatment of more than one disease state and meets the criterion for the stability and effective seroprotection for each of the said immunogenic components. It is further provided that the polysaccharide-carrier protein conjugate vaccine composition is a monovalent bulk polysaccharide-carrier protein conjugate or multivalent polysaccharide-carrier protein conjugate or a drug product comprising of polysaccharide-carrier protein conjugates. In one of the aspects, monovalent bulk polysaccharide-carrier protein conjugate is a component stored prior to formulation. In other aspect monovalent bulk polysaccharide-carrier protein conjugate vaccine is a drug product comprising of polysaccharide-carrier protein conjugate and the excipient and stabilizers. In other aspect multivalent polysaccharide-carrier protein conjugate is a mixture of one or more different types of polysaccharide-carrier protein conjugates and is free of excipient and stabilizers. In another aspect drug product of multivalent polysaccharide-carrier protein conjugate is a mixture of one or more different types of polysaccharide-carrier protein conjugates along with the excipient and stabilizers. In one of the aspects of the present embodiment, the polysaccharide-carrier protein conjugate vaccine composition may additionally comprise of antigen of viral origin, recombinant proteins, inactivated and/or attenuated bacteria.

An embodiment of the present disclosure provides a polysaccharide-carrier protein conjugate vaccine composition is a fully liquid vaccine composition and/or a lyophilized vaccine composition. As used herein the terms "Freeze-drying" or "lyophilize" or "lyophilization" involves lyophilization and refers to the process by which a suspension is frozen, after which the water is removed by sublimation at low pressure. As used herein, the term "sublimation" refers to a change in the physical properties of a composition, wherein the composition changes directly from a solid state to a gaseous state without becoming a liquid. In one of the aspect of said embodiment it is provided that said liquid or lyophilized vaccine composition may comprise of a stabilizer combination selected from a) 2 to 5% (w/v) Trehalose, 0.25 to 0.75% sodium citrate; b) 2 to 5% (w/v) Sucrose and 0.25 to 0.75% sodium citrate; c) 2 to 5% (w/v) Sucrose,2 to 5% (w/v) Lactose and 0.25 to 0.75% sodium citrate; d) 2 to 5% (w/v) Trehalose ,2 to 5% (w/v) Lactose and 0.25 to 0.75% sodium citrate; e) Sucrose, Potassium dihydrogen phosphate, Sodium dihydrogen phosphate monohydrate, Disodium phosphate dihydrate, sodium chloride, water for injections; f) sodium phosphate buffered isotonic sodium chloride solution; g) Sucrose, Trometamol, Sodium chloride, Water for injections; h) sodium chloride, sodium acetate; i) Sucrose and Mannitol. Another aspect of the said embodiment it is provided that said vaccine composition may further comprise a buffer selected from Tris and phosphate.

In one of the aspects of said embodiment it is provided that said liquid or lyophilized vaccine composition may comprise of one or more selected 2-phenoxyethanol, Benzethonium chloride (Phemerol), Phenol, m-cresol, Thiomersal, Formaldehyde, paraben esters (e.g. methyl-, ethyl-, propyl- or butyl- paraben), benzalkonium chloride, benzyl alcohol, chlorobutanol, p-chlor-m-cresol, or benzyl alcohol or a combination thereof. It is also understood that the preservatives may be used in case of multi-dose presentations of vaccine composition, whereas single dose vaccine formulations may be devoid of preservatives.

An embodiment of the present disclosure provides a polysaccharide-carrier protein conjugate vaccine composition, wherein the polysaccharide is an immunogenic component and is derived from a microorganism. In one of the aspect of the present embodiment, the polysaccharide is derived from a gram negative and/or gram positive microorganism. It is further provided that polysaccharide is one or more polysaccharide derived from the group comprising of but not limited to Helicobacter pylori, Chlamydia pneumoniae, Chlamydia trachomatis, Ureaplasma urealyticum, Mycoplasma pneumoniae, Staphylococcus spp., Staphylococcus aureus, Streptococcus spp., Streptococcus pyogenes, Streptococcus agalactiae, Streptococcus pneumoniae, Streptococcus viridans, Enterococcus faecalis, Neisseria meningitidis, Neisseria gonorrhoeae, Bacillus anthracis, Salmonella spp., Salmonella typhi, Salmonella paratyphi, Salmonella typhimurium, Salmonella enteritidis Vibrio cholerae, Pasteurella pestis, Pseudomonas aeruginosa, Campylobacter spp., Campylobacter jejuni, Clostridium spp., Clostridium difficile, Mycobacterium spp., Mycobacterium tuberculosis, Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Hemophilus influenzae, Escherichia coli, Shigella spp., Ehrlichia spp., and Rickettsia spp. Polysaccharides of Streptococcus pneumoniae type 1 , 2, 3, 4, 5, 6A, 6B, 7F, 8, 9N, 9V, 10A, 11A, 12F, 14, 15B, 15C, 17F, 18A, 18B, 18C, 18F, 19F, 19A, 20, 22F, 23F, 35B, 38, 45 and 33F, Polysaccharides of Meningococcal serogroup A, B, C, D, W135, X, Y, Z, 29E, H. influenzae type b, etc.

In one of the aspects of the present embodiment, polysaccharide may be conjugated to one or more carrier protein selected from group comprising of but not limited to CRM197, diphtheria toxoid, Tetanus toxoid, Neisseria meningitidis outer membrane complex, fragment C of tetanus toxoid, pertussis toxoid, protein D of H. influenzae, E. coli LT, E. coli ST, exotoxin A from Pseudomonas aeruginosa, outer membrane complex c (OMPC), porins, transferrin binding proteins, pneumolysin, pneumococcal surface protein A (PspA), pneumococcal surface adhesin A (PsaA), PhtA, PhtB, PhtE, pneumococcal PhtD, pneumococcal surface proteins BVH-3 and BVH-11, M. catarrhalis uspA, protective antigen (PA) of Bacillus anthracis and detoxified edema factor (EF) and lethal factor (LF) of Bacillus anthracis, ovalbumin, keyhole limpet hemocyanin (KLH), C5a peptidase group A or group B Streptococcus, human serum albumin, bovine serum albumin (BSA), purified protein derivative of tuberculin (PPD), Cholera toxin subunit B, fHbp, Por A and Por B . It is further provided that the skilled person in the art, the vaccine may comprise of conjugate(s) wherein afore-mentioned polysaccharides are conjugated to afore-mentioned carrier proteins in any combination.

In another aspect of the present embodiment, polysaccharide-carrier protein conjugate vaccine composition comprises of a polysaccharide derived from Neisseria meningitidis. The said polysaccharide may be derived from Neisseria meningitidis serogroup A, C, W, Y and X, and further conjugated to carrier protein selected from CRM197 and/or Tetanus toxoid. N. meningitidis serotype X strains selected from M9601, M9592, M9591, 247X, M9554, M8210 and M2526, 5967 strain (ST 750), most preferably to "M8210".

In preferred aspect of the said embodiment, the polysaccharide-carrier protein conjugate vaccine composition comprises of a polysaccharide derived from Neisseria meningitidis serogroup A conjugated to Tetanus toxoid; Neisseria meningitidis serogroup C conjugated to CRM197; Neisseria meningitidis serogroup W conjugated to CRM197; Neisseria meningitidis serogroup Y conjugated to CRM197; and/or Neisseria meningitidis serogroup X conjugated to Tetanus toxoid.

In another aspect of the said embodiment, Neisseria meningitidis vaccine composition (liquid/lyophilized) comprises of each conjugate having concentration of 5-15 microgram per millilitre, along with excipient including 10-100 milligram per millilitre of Sucrose, 1-10 milligram per millilitre of Sodium citrate dehydrate, and 0.1 - 2 milligram per millilitre of Tris. The vaccine composition may additionally comprise one or more of 10-50 milligram per millilitre of Mannitol, 1-20 milligram per millilitre of Sodium chloride and/or 0.1 - 20 milligram per millilitre of 2-Phenoxyethanol (as preservative). It is very well understood that the concentration of excipient may be designed in a way to obtain the desired osmolality of vaccine composition.

In another aspect of the present embodiment, polysaccharide-carrier protein conjugate vaccine composition comprises of a polysaccharide derived from Streptococcus Pneumoniae.

The said polysaccharide may be derived from serogroup 1, 2, 3, 4, 5, 6A, 6B, 7F, 8, 9N, 9V, 10A, 11A, 12F, 14, 15B, 17F, 18C, 19F, 19A, 20, 22F, 23F, 35B, 38, 45 and 33F and further conjugated to one or more carrier protein selected from CRM197, Tetanus Toxoid, Diphtheria Toxoid, Protein D, Pneumolysin and/or PsaA.

In a preferred aspect of present embodiment, the polysaccharide - carrier protein conjugate vaccine composition comprises of a 10 or more Streptococcus Pneumoniae polysaccharide conjugated to one or more carrier proteins selected from Diphtheria Toxoid, Tetanus toxoid and CRM197.

In another aspect of the present embodiment, polysaccharide-carrier protein conjugate vaccine composition comprises of a polysaccharide derived from Salmonella Spp. The said salmonella spp includes one or more of S. typhi, Salmonella enterica serovar Paratyphi (S. Paratyphi) A and B, Salmonella enterica serovar Typhimurium (S. Typhimurium) and Salmonella enterica serovar Enteritidis (S. Enteritidis). The said polysaccharides may be conjugated to one or more carrier proteins selected from the group comprising of Tetanus toxoid, CRM197 and/or Diphtheria toxoid.

In one of the embodiments of the present disclosure, the vaccine composition comprises of a polysaccharide-protein conjugates having maximum molecular size in the range of 10 - 10000 kDa. The polysaccharide may be subjected to sizing before conjugation. In another aspect of the present embodiment, the polysaccharide is subjected to sizing using mechanical or chemical means before conjugation. The polysaccharide may be subjected to mechanical sizing methods including homogenization, sonication, microfluidization and high pressure cell disruption; and/or chemical sizing methods like use of sodium acetate, acids/low pH treatment or bases/high pH treatment.

In a preferred aspect of this embodiment, the vaccine composition is a pentavalent vaccine composition comprising of polysaccharides derived from Neisseria meningitidis serogroup A and X conjugated to Tetanus toxoid; and serogroup C, W, & Y conjugated to CRM197. The polysaccharide may be conjugated to a carrier protein using reductive amination, cyanylation, carbodiimide conjugation chemistry.

The composition may additionally comprise of excipients like Sucrose, Mannitol, Sodium citrate dehydrate, Tris, 2-phenoxyethanol as preservative. It is further provided that the total molar mass/ molecular weight would add value to help determining & maintaining the consistency of lot-to-lot manufacturing of vaccine composition.

The geographical source of the strains is as follows:

| Name of the Organism | Strain Designation | Source of Strain |
|---|---|---|
| Neisseria meningitidis A | M1027 | SynCo Biopartners (Netherlands) |
| Neisseria meningitidis C | C11(60E) | CBER/FDA, USA |
| Neisseria meningitidis W | S877 | CBER/FDA, USA |
| Neisseria meningitidis Y | M10659 | CDC, USA |
| Neisseria meningitidis X | M8210 | CBER/FDA, USA |
| Unconjugated carrier protein, i.e. CRM197 or TT. CRM197 was derived from Recombinant Strain CS463-003 (MB101) of Pseudomonas fluorescens from Pfenex USA. TT was derived from Clostridium Tetani (Harvard No 49205) from NVI, Netherland. | | |

In an embodiment of the present disclosure, optimized assay is provided to provide data with respect to molecular size distribution & molar mass distribution correlating individual/monovalent bulk conjugates and the multivalent vaccine composition (drug product).

In an embodiment of the present disclosure, it is provided to design a vaccine composition with a pattern of molecular size distribution & molar mass distribution of the vaccine composition.

In an aspect of the said embodiment, it is provided to design a vaccine composition with specified range (%) of average molecular weight (AMW) with stability, immunogenicity and potency/efficacy of the vaccine composition.

In other aspect of the said embodiment, it is provided to design a vaccine composition with specified pattern/range of High molecular weight (HMW) and Low molecular weight (LMW) with stability, immunogenicity and potency/efficacy of the vaccine composition.

In another aspect of the said embodiment, it is provided to design a vaccine composition with pattern/range of average molecular weight (AMW), high molecular weight (HMW) and Low molecular weight (LMW) with stability, immunogenicity and potency/efficacy of the vaccine composition.

In a further aspect of the present embodiment, it is further provided to design a vaccine composition without interference of excipient in a vaccine composition.

In another aspect of the said embodiment, the said vaccine composition is provided to be analyzed for determining characteristics/attributes of each component present in a vaccine composition using the assay provided in the present disclosure.

In an embodiment of the present invention, multivalent Neisseria meningitidis polysaccharide carrier protein conjugate vaccine composition is provided. It is further provided that said vaccine composition comprises of specified amount of Average molecular weight components with optimum immunogenicity, potency and stability. It is also provided that said vaccine composition comprises of specified amount of Low molecular weight and High molecular weight components with optimum immunogenicity, potency and stability. It is also provided that a vaccine composition having specified correlation between ratio of Average molecular weight; Low molecular weight and High molecular weight components provides optimum immunogenicity, potency and stability.

**Technical Advantages:**

The present disclosure described herein above has several technical advantages including, but not limited to, the realization of:

- The present disclosure provides a process for assaying stability of liquid and lyophilized vaccine composition.
- The present disclosure provides an assay for stability analysis of monovalent and/or multivalent polysaccharide protein conjugate vaccine.
- The present disclosure provides an assay for stability analysis of multivalent polysaccharide protein conjugate vaccine wherein analysis of individual/each polysaccharide protein conjugate is not feasible.
- The present disclosure provides an assay for stability analysis of vaccine composition and helps to elucidate the characteristics/attributes of components /molecules present in different dose presentations and formulations, in terms of molecular weight/molar mass and molecular size to further enable assessment of stability of the vaccine composition in a single assay.
- The present disclosure provides an assay for stability analysis of polysaccharide protein conjugate vaccine comprising one or more type of polysaccharide; and one or more carrier proteins.
- The present disclosure provides an assay that maintains the integrity of the molecule/component of vaccine composition, and thereby helps relate stability and immunogenicity of the vaccine composition.
- The present disclosure provides a process for size exclusion chromatography and Multi-angle light scattering (MALS) assay; and the said process is non-destructive, maintains integrity of components/molecules, independent of the presence of excipients; and avoids use of harsh conditions thereby providing true presentation of the components/-molecules in a vaccine composition.
- The present disclosure provides an assay for stability analysis (lot to lot) of polysaccharide protein conjugate vaccine with respect to molar mass distribution and/or molecular size distribution, and data can be utilized for quality control during storage and batch characterization.
- The present disclosure provides a vaccine composition with optimized molar mass distribution and/or molecular size distribution with improved stability and immunogenicity of the vaccine composition.
- The present disclosure provides a method for profiling of conjugates based on molecular size and/or molar mass in a multivalent N. Meningitidis conjugate vaccine (ACWYX) drug product using SEC-HPLC-MALS; showing correlation between Molecular Size Distribution (SEC-MALS) and Stability [comprising profiling in terms of Average Molecular Weight/AMW, High Molecular
- Weight/HMW, Low Molecular Weight/LMW molecules] which contribute to determining lot-to-lot consistency, stability of multivalent drug product contributing to integrity, degradation and aggregation profile of multivalent drug product and its ingredients] wherein such molecular size distribution (measurement & profiling of a multivalent in terms of AMW, HMW, LMW) is an effective alternative method (in addition to free polysaccharide measurement) to assess stability of multivalent conjugate mix ACWYX (drug product)
- The present disclosure provides a method for measuring aggregation profile & molecular size distribution of a multivalent drug product mix and is done in the presence of two carrier proteins, free polysaccharides and excipient, without any interference of said components.

- The present disclosure provides a method that utilizes set of three columns [Shodex 807 + Shodex 806/mix bead column SB806M + TSK gel 5000/6000 PwxL + with guard column] in a series, using Phosphate buffer as mobile phase along with optimized flow rate, time, temperature and detectors [UV; RI; & MALS].
- The present disclosure provides a stability profile of vaccine compositions stored at -20, 2-8, 25, 37, 40 & 50-55/60 Degree Celsius.

EXAMPLES:

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skilled in the art should, in light of the present disclosure, appreciate that many changes can be made in the specific embodiments which are disclosed and still obtain a like or similar result without departing from the scope of the invention.

**Example 1: Lyophilized and Liquid composition / formulation**

Following lyophilized (Table 1) and liquid (Table 2) polysaccharide protein conjugate vaccine was considered and used further for analysis.

Table 1: Lyophilized vaccine post reconstitution with 0.9% Sodium Chloride:

| Ingredient | Category | Quantity per mL-Single Dose | Quantity per mL-5 Dose |
|---|---|---|---|
| Men A-TT Conjugate | Active | 10 micrograms | 10 micrograms |
| Men C-CRM Conjugate | Active | 10 micrograms | 10 micrograms |
| Men Y-CRM Conjugate | Active | 10 micrograms | 10 micrograms |
| Men W-CRM Conjugate | Active | 10 micrograms | 10 micrograms |
| Men X-TT Conjugate | Active | 10 micrograms | 10 micrograms |
| Sucrose | Excipient | 23.80 milligrams | 4.84 milligrams |
| Sodium citrate dihydrate | Excipient | 3.96 milligrams | 0.80 milligrams |
| Tris | Excipient | 0.96 milligrams | 0.196 milligrams |
| Sodium Chloride | Excipient | 9 milligrams | 9 milligrams |
| Dose Volume: 0.5mL | | | |

Table 2: Liquid Formulation

| Ingredient | Category | Quantity per mL-Single Dose | Quantity per mL-5 Dose |
|---|---|---|---|
| Men A-TT Conjugate | Active | 10 micrograms | 10 micrograms |
| Men C-CRM Conjugate | Active | 10 micrograms | 10 micrograms |
| Men Y-CRM Conjugate | Active | 10 micrograms | 10 micrograms |
| Men W-CRM Conjugate | Active | 10 micrograms | 10 micrograms |
| Men X-TT Conjugate | Active | 10 micrograms | 10 micrograms |
| Sucrose | Excipient | 23.80 to 30 milligrams | 23.80 to 30 milligrams |
| Sodium citrate dihydrate | Excipient | 3.96 to 5 milligrams | 3.96 to 5 milligrams |
| Tris | Excipient | 0.96 to 1.21 milligrams | 0.96 to 1.21 milligrams |
| Sodium chloride | Excipient | 4.5 milligrams | 4.5 milligrams |
| Preservative (2-PE) | Excipient | No | 5 to 15 milligrams |

**Example 2: Use of three chromatography columns in series**

Since, the concept is the analysis of formulation containing multiple serogroups along with multiple carrier proteins i.e. a pentavalent (multivalent) mix, it would not be scientifically as well as analytically judicious to apply or use the single column method, which has limitation with respect to resolution or separation ability of molecules noting its exclusion limits. Therefore, the comparison between single column Vs multiple columns becomes not applicable in this case. Hence, the complete development was conducted on series of columns i.e. three columns in series to understand the behaviour of molecule from the starting phase to establish the methodical correlation with respect to changes, which may happen during the study.

Hence, since beginning accepting the complex nature of formulation and challenges which may appear with respect to structural change in the molecule over the period of time during stability studies the requirement of dynamic column separating range (exclusion limit i.e. separation capacity) was necessary and needed.

Further, due to non-availability of a single column, which can provide the envisaged wide range of separating capacity, a unique column set up was assembled by attaching three columns in a series. The wise selection of each individual column was considered which would have ability to separate out not only the monovalent but multivalent formulation samples including stability studies sample exposed at various temperatures when connected in series.

**Example 3: Process**

**Step 1: MATERIAL & EQUIPMENT:**

- HPLC machine with UV, RI and MALS detector (Eg. Agilent/Wyatt)
- Test tube stand/rack (Eg. Tarson)
- Variable volume Micropipettes and tips (Eg. Eppendorf/Brand)
- Variable volume beakers and measuring cylinder (Eg. Borosil)
- Variable volume micro centrifuge tube (Eg. Eppendorf/Tarson)
- Variable volume bottles (Eg. Schott/Borosil)
- Weighing balance (Eg. Sartorius)
- Vials for HPLC (Eg. Waters/ Thermo)
- Solvent filtration unit with receiving flask (Eg. Pall/Millipore)
- pH meter (Eg.Orion)
- Sonicator (Eg. Soltec - Sonica3300EP03)
- Laboratory vortex mixer (Eg. Spinix)
- 0.1 $\mu$ filters for buffer filtration (Eg. Pall/Millipore)
- Centrifugal devices 10 KDa (Filters) (Eg. Millipore/Amicon)
- Centrifuge (e.g. Thermo)

**Step 2: CHEMICALS AND REAGENTS:**

- WFI/Milli Q Water (WFI/MQW)
- Potassium dihydrogen orthophosphate (Eg. Fisher Scientific 13405)
- Di-Sodium hydrogen orthophosphate anhydrous (Eg. Fisher Scientific 15825)
- Sodium Chloride (Eg. Fischer Scientific 15918).
- Sodium azide (Eg.Sigma S8032).
- Deoxyribonucleic acid sodium salt from salmon testes (Salmon DNA-S.DNA) (Eg. Sigma Aldrich D1626)
- Ethylene glycol (EG) (Sigma 85978)
- Bovine serum albumin (BSA) 2 mg/mL (e.g. Thermo)
- Trizma (Tris) (Sigma T6066)

**Step 3: Procedure:**

- Prime the HPLC pump and line with the filtered mobile phase i.e. PBS.

- Set the flow rate to 0.8 mL/min and connect to the column inlet, ensuring that flow is in the direction indicated by the arrow on the column. Connect column outlet to flow cell. Make the column connection and pass sufficient (At least 2 column) volumes through the system at 0.8 mL/min while observing the back pressure.
- The column can be kept on equilibration overnight at reduced flow of 0.1 mL/min.
- Select the project and open it. Start the MALS and RI detectors.
- Prime the HPLC system with PBS and connect the columns.

**Step 4: Procedure:**

Sample Preparation (Pre-treatment):

Vaccine vial was reconstituted in 0.5-1.0 mL of 10mM Tris buffer. The 10 kDa Amicon ultra-15 centrifugal filter device was assembled, and 15 mL of Milli Q water was added. The filter device was centrifuged at 4400 g for 30 min as prewash. The Milli Q water was discarded, and reconstituted sample was added in sample reservoir. Volume was made up to 15 mL with 10 mm Tris buffer. The sample was centrifuged at 4400 g for 1 hr. ~100 μL of the sample was collected in microfuge tube and final volume was made to 500 μL. The sample was used for further analysis in HPLC.

Mobile Phase with concentration:

Phosphate buffer saline (PBS): 1800 mL MQW was taken in 2000mL bottle. 2.87 g di-sodium hydrogen orthophosphate, 1.0 g of potassium dihydrogen orthophosphate and 23.38 g of sodium chloride were weighed and added. The bottle was kept on magnetic stirrer with the magnetic bead into it. The component was dissolved by stirring it on magnetic stirrer. The pH was adjusted with 50 % NaOH to pH 7.4. The final volume was made up to 2000 ml with MQW. The mobile phase was filtered using 0.1μ filters and was stored at room temperature.

Table 3, 4 and 5 provide details with respect to process parameters, column details and HPLC make/Model

Table 3: Process parameters

| Parameters | Description |
|---|---|
| pH | 7.4 ±0.2 |
| Column Temperature: | 30°C ± 5°C |
| Sample temperature | 5 ± 3°C |
| Time period/injection (Run Time): | 60-70 min |
| Column details - Main column | Shodex OHPAK SB 807 HQ (Column- ID: 7.8mm and Length: 30 cm, Part No. F6429105)<br>Shodex OHPAK SB 806 HQ (Column- ID: 7.8mm and Length: 30 cm, Part No. F6429105)<br>G6000 PWXL (Column- ID: 7.8mm and Length: 30 cm, Part No. - 0008023) |
| Column details - Guard column | Shodex OHPAK SB-G-6B |
| Detector 1 | UV Detection Wavelength |
| Detector 2 | Refractive index detector |
| Detector 3 | MALS (Multi Angle light scattering) |
| Flow Rate: | 0.50 to 0.80 mL/min |
| Elution Mode : | Isocratic |
| Mobile Phase | Phosphate buffer saline (PBS) 7.4 ±0.1 |
| System stability | Salmon DNA and Ethylene Glycol |

Table 4: Column details

| Column | Particle size | Dimension | Material of resin | Exclusion limit (Kilodalton) |
|---|---|---|---|---|
| Shodex OHPAK SB 807 HQ | 35 $\mu$m | Column- ID: 7.8mm and Length: 30 cm | Poly (hydroxymethacrylate)-type porous particle. | 500000 |
| Shodex OHPAK SB 806 HQ) | 13 $\mu$m | Column- ID: 7.8mm and Length: 30 cm | Poly (hydroxymethacrylate)-type porous particle. | 20000 |
| G6000 PWXL | 13 $\mu$m | Column- ID: 7.8mm and Length: 30 cm | Hydroxylated Polymethacrylate | 8000 |
| Guard column (Shodex OHPAK SB-G-6B) | 13$\mu$m | Size (mm) I.D. x Length: 6.0 x 50 | Polymer-based packed column* | Not Applicable |
| (*Polymer-based packed column - For e.g. polyhydroxymethacrylate based material, etc.) | | | | |

Table 5: HPLC make/Model

| Details | Make | Model |
|---|---|---|
| HPLC machine with UV detector | Agilent | 1260 Bio Quat. Pump |
| RI detector | Wyatt | Optilab T-rex (WTREX-11) |
| MALS detector | Wyatt | DAWN Helios II (WH2-11) |

Sample injection:

A suitable mobile phase was used as mentioned above and the vaccine was reconstituted and run into the same mobile phase along with mobile phase blank. The Blank injection showed the non-interference of the mobile phase components in the analysis.

Calculation formula:

To arrive at the distribution cut the following mathematical calculation is considered

$$K_D\,(0.5) = V_e\text{-}V_o \, / \, V_t\text{-}V_0$$

$$V_e = 0.5\ X\ (V_t\text{-}V_o) + V_o$$

Where,

$K_D$ = Distribution coefficient.
$V_e$ = Elution volume
$V_o$ = Void volume
$V_t$ = total bed volume

An elution volume ($V_e$) is where the peak is cut to get percent (%) molecular size distribution.

For HMW: $V_e$ is 0.1 $K_D$
For LMW: $V_e$ is 0.5 $K_D$
For AMW: Peak start to peak end is considered.

**Example 4: Determination of HMW, AMW, LMW**

To characterize the sample, firstly the multiple i.e. 1D and 5D formulation samples were analyzed by the optimum method to get an information on the average molecular weight (AMW) of the lyophilized multivalent (pentavalent) mix. In the next stage, we analyzed the multivalent (pentavalent) liquid formulation to obtain the information on average molecular weight. Then a comparative analysis was run to understand the sample behaviour in Lyophilized Vs Liquid as shown in the Table 6.

Table 6: Comparative analysis of sample behaviour in Lyophilized Vs Liquid

| Presentation | Batch No. | Pentavalent MenFive Liquid formulation | Pentavalent MenFive Lyophilized formulation |
|---|---|---|---|
| 1 Dose | 2349T001 | 5051 | 5420 |
| | 2349T003 | 5430 | 5354 |
| 5 Dose | 2359T001 | 5269 | 6109 |
| | 2359T002 | 5283 | 6410 |
| | 2359T003 | 5217 | 6659 |

The data presented below indicated that the AMW for lyophilized formulation is more as compare to liquid. Whereas the 1 dose formulation looks alike in Lyophilized and Liquid. This triggered to measure and characterizes the same sample on molecular weight scale/ruler.

Based on the AMW and distribution of the molecules with respect to molar mass, the following presentation cum information about the sample was obtained which gives an information about presence and percentage of HMW, AMW and LMW molecules (Table 7 and 8). The chromatogram obtained provides presence 100% molecules. Further the peak is split/cut to obtain HMW, Main peak and LMW which finally resulted into the percent contribution of molecules per split as provide in Tables 7 and 8.

Table 7: Presence and percentage of HMW, AMW and LMW molecules in Lyophilized vaccine sample

| Distribution | Molecular weight Scale (kDa) | Lyophilized sample | % Contribution of the molecules |
|---|---|---|---|
| HMW | 17000 to 19000 | 1 | 9 |
| | 15000 to 17000 | 3 | |
| | 13000 to 15000 | 5 | |
| AMW | 11000 to 13000 | 7 | 88 |
| | 10000 to 11000 | 4 | |
| | 9000 to 10000 | 4 | |
| | 8000 to 9000 | 5 | |
| | 7000 to 8000 | 5 | |
| | 6000 - 7000 | 6 | |
| | 5000 to 6000 | 7 | |
| | 4000 to 5000 | 9 | |
| | 3000 to 4000 | 13 | |
| | 2000 to 3000 | 28 | |
| LMW | 1000 to 2000 | 3 | 3 |
| | 200 to 1000 | 0 | |

Table 8: Presence and percentage of HMW, AMW and LMW molecules in Lyophilized vaccine sample

| Distribution | Molecular weight Scale (kDa) | Liquid sample | % Contribution of the molecules |
|---|---|---|---|
| HMW | 17000 to 19000 | 0 | 14 |
| | 15000 to 17000 | 0 | |
| | 13000 to 15000 | 3 | |
| | 11000 to 13000 | 7 | |
| | 10000 to 11000 | 4 | |
| AMW | 9000 to 10000 | 4 | 85 |
| | 8000 to 9000 | 5 | |
| | 7000 to 8000 | 5 | |
| | 6000 - 7000 | 5 | |
| | 5000 to 6000 | 7 | |
| | 4000 to 5000 | 8 | |
| | 3000 to 4000 | 12 | |
| | 2000 to 3000 | 17 | |
| | 1000 to 2000 | 22 | |
| LMW | 200 to 1000 | 0 | 1 |

Above both the tables (Table No. 7 and 8) provides information about the dominating population of molecules present in the mixed sample.

Table 9: The following table provides the summarize information about the presence of HMW, AMW and LMW

| Presentation | Lyophilized | Liquid |
|---|---|---|
| HMW (kDa) | 13000 to 19000 | 10000 to 19000 |
| AMV (kDa) | 2000 to 11000 | 1000 to 10000 |
| LMW (kDa) | 200 to 2000 | 200 to 1000 |

**Example 5: Method Validation**

Since the standards are not required for the estimation of molecular size or weight, the linearity, range, becomes not applicable. In addition, as per the standard guidance, the only parameter becomes applicable in case of molecular size or estimation is precision (intraday and interday), is established and result mentioned confirms the accuracy of the test. The precision data shown in Tables 10 and 11 is presented below-

Table 10: Precision data of 1D sample

| Presentation | Date | Molecular weight (kDa) | | | |
|---|---|---|---|---|---|
| | | HMW | Main peak | LMW | AMW |
| 1 Dose | Analysis 1 | 17090 | 6449 | 4525 | 6551 |
| | Analysis 2 | 13370 | 5801 | 2485 | 6313 |
| | Analysis 3 | 14910 | 6119 | 3661 | 6157 |
| | %CV | 12 | 5 | 29 | 3 |

Table 11: Precision data of 1D sample

| Presentation | Date | Molecular weight (kDa) | | | |
|---|---|---|---|---|---|
| | | HMW | Main peak | LMW | AMW |
| 5 Dose | Analysis 1 | 16580 | 6026 | 4177 | 6107 |
| | Analysis 2 | 13870 | 6180 | 4939 | 5772 |
| | Analysis 3 | 16250 | 5826 | 2822 | 5767 |
| | %CV | 9 | 3 | 27 | 3 |

## Example 6: Stability studies

I. Stability studies of MenFive (Lyophilized) vaccine 1 Dose and 5 Dose at 25°C, 40°C, 60°C of 1 month compared with initial 2-8°C

Table 12: Stability Data of 1 Dose MenFive (Lyophilized vaccine) at 25°C, 40°C, 60°C of 1 month compared with initial 2-8°C

| MenFive (Lyophilized vaccine) | Stability Data: 1 Dose | | | |
|---|---|---|---|---|
| | Weights in Kilodalton (kDa) | | | |
| | HMW | Main Peak | LMW | AMW (Total Peak) |
| 2-8°C (Initial) | 14910 | 6119 | 3661 | 6157 |
| 25°C (at 1 Month) | 13520 | 6194 | 3990 | 6256 |
| 40°C (at 1 Month) | 15920 | 6235 | 3319 | 6288 |
| 60°C (at 1 Month) | 12790 | 5908 | 2942 | 5915 |

**Observations:**

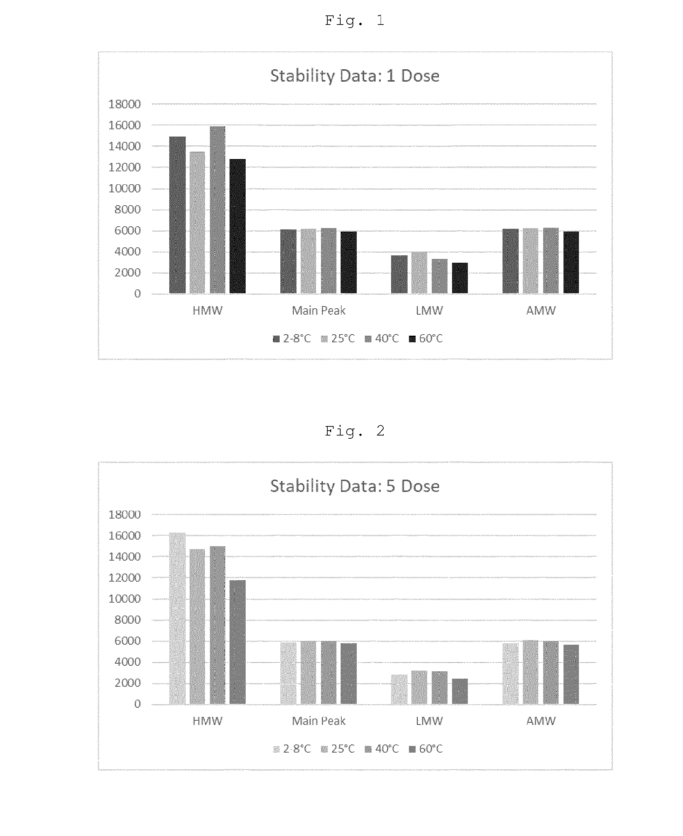

From Table 12 and Figure 1; following observations were found.
For HMW molecules: After one-month analysis,

- At temperature condition which is higher than initial temperature i.e. at 25°C, presence of HMW molecules decreases which is indicated by decrease in weight (Kilodalton) of the molecules.
- At temperature condition 40°C, the HMW molecules increases
- At temperature condition 60°C, the HMW molecules decreases up to 12790.

For LMW molecules: After one-month analysis,

- There are no major changes in the presence of LMW molecules at all temperature conditions 25°C, 40°C and 60°C as compared to the initial (2-8°C) temperature condition.

For AMW molecules: After one-month analysis,

- There are no major changes in the presence of AMW molecules at all temperature conditions 25°C, 40°C and 60°C as compared to the initial (2-8°C) temperature condition.

**Conclusion:**

- Presence of HMW molecules indicate the occurrence of aggregation. As compared to the initial/control readings in the above table, decrease in molecular weight of HMW molecules as compared to the initial/control readings indicates aggregation is reduced at one month, 25°C; one month, 40°C increase in molecular weight of HMW molecules as compared to the initial/control readings indicates aggregation is increased; whereas at one month, 60°C decrease in the molecular weight of HMW molecules indicate decrease in aggregation.

- As compared to the initial/control readings in the above table, there is no change in the molecular weights (Kilodalton) of LMW molecules which indicate that there is no any degradation.
- As compared to the initial/control readings in the above table, there is no change in the molecular weights (Kilodalton) of AMW molecules which indicate the stable nature of the molecule.

Table 13: Stability Data of 5 Dose MenFive (Lyophilized vaccine) at 25°C, 40°C, 60°C of 1 month compared with initial 2-8°C

| MenFive (Lyophilized) | Stability Data: 5 Dose | | | |
|---|---|---|---|---|
| | Weights in Kilodalton (kDa) | | | |
| | HMW | Main Peak | LMW | AMW |
| 2-8°C (Initial) | 16250 | 5826 | 2822 | 5767 |
| 25°C (at 1 Month) | 14720 | 6052 | 3184 | 6093 |
| 40°C (at 1 Month) | 14980 | 6067 | 3156 | 6015 |
| 60°C (at 1 Month) | 11800 | 5762 | 2419 | 5665 |

**Observations:**

From Table 13 and Figure 1; following observations were found.

For HMW molecules: After one-month analysis,

- At temperature condition which is higher than initial (control) temperature i.e. at 25°C, presence of HMW molecules decreases which is indicated by decrease in weight (Kilodalton) of the molecules.
- At temperature condition 40°C, the HMW molecules increase when compared to the results of 25°C but is found decreased when compared to the initial (control) temperature
- At temperature condition 60°C, the HMW molecules decreases in the large quantity up to 11800.

For LMW molecules: After one-month analysis,

- There are no major changes in the presence of LMW molecules at all temperature conditions 25°C, 40°C and 60°C as compared to the initial (2-8°C) temperature condition.

For AMW molecules: After one-month analysis,

- There are no major changes in the presence of AMW molecules at all temperature conditions 25°C, 40°C and 60°C as compared to the initial (2-8°C) temperature condition.

**Conclusion:**

- Presence of HMW molecules indicate the occurrence of aggregation. As compared to the initial/control readings in the above table, decrease in molecular weight of HMW molecules indicates aggregation is reduced at one month, 25°C; at one month, 40°C decrease in the molecular weight of HMW molecules as compared to the initial/control readings indicate decrease in aggregation; at one month, 60°C decrease in the molecular weight of HMW molecules as compared to the initial/control readings indicate decrease in aggregation.
- As compared to the initial/control readings in the above table, there is no change in the molecular weights (Kilodalton) of LMW molecules which indicate that there is no any change in the amt of free polysaccharide or protein molecules present in the vaccine sample.
- As compared to the initial/control readings in the above table, there is no change in the molecular weights (Kilodalton) of AMW molecules which indicate that there is no any change in the amt of free polysaccharide or protein molecules present in the vaccine sample.

II. Comparison between Lyophilized and Liquid vaccine of 1 Dose and 5 Dose at 2-8°C (initial), 25°C, 40°C, 60°C respectively:

Table 14: Comparison between Lyophilized and Liquid vaccine of 1 Dose at 2-8°C (initial), 25°C, 40°C, 60°C respectively

| | 1 Dose | | | |
|---|---|---|---|---|
| | Comparison between Lyophilized Vs Liquid Vaccine | | | |
| Temperature | HMW | Main Peak | LMW | AMW |
| 2-8°C (Lyo) | 14910 | 6119 | 3661 | 6157 |
| 2-8°C (Liq) | 14440 | 6210 | 3749 | 6181 |
| Temperature | HMW | Main Peak | LMW | AMW |
| 25°C (Lyo) | 13520 | 6194 | 3990 | 6256 |
| 25°C (Liq) | 15430 | 5959 | 2088 | 5818 |
| Temperature | HMW | Main Peak | LMW | AMW |
| 40°C (Lyo) | 15920 | 6235 | 3319 | 6288 |
| 40°C (Liq) | 14410 | 5683 | 1238 | 5269 |
| Temperature | HMW | Main Peak | LMW | AMW |
| 60°C (Lyo) | 12790 | 5908 | 2942 | 5915 |
| 60°C (Liq) | 20870 | 4917 | 598 | 3826 |

**Observations:**

Table 14 focuses on the presentation of molecules in terms of molecular weight.

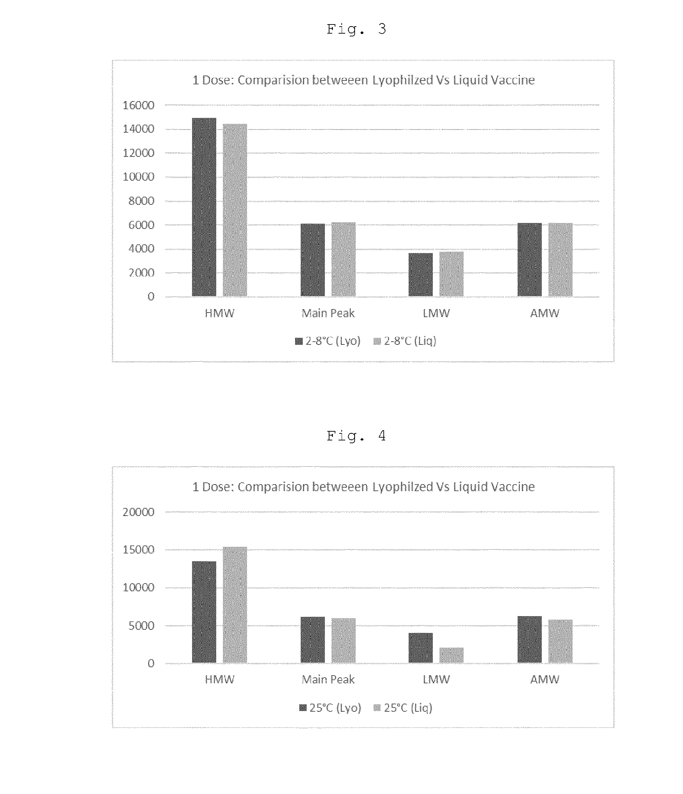

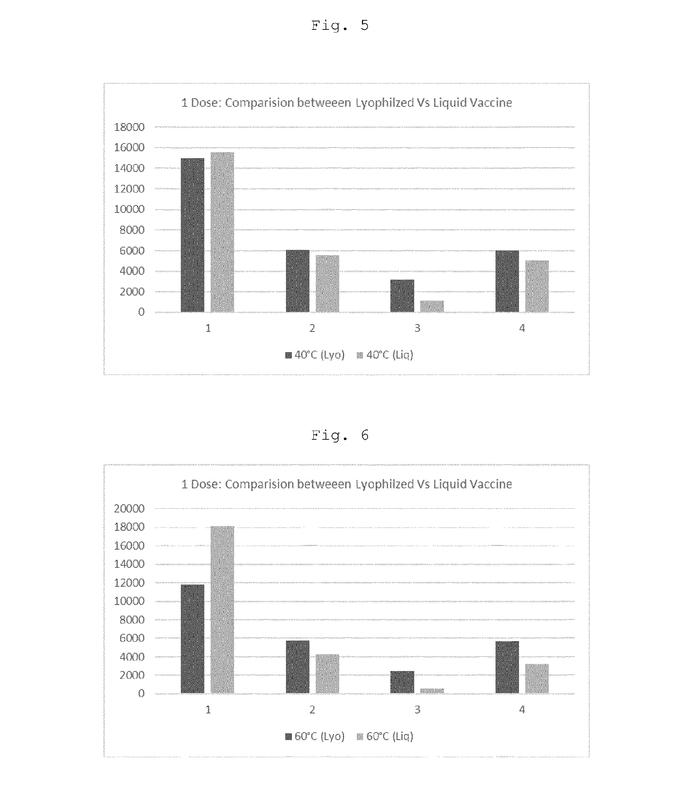

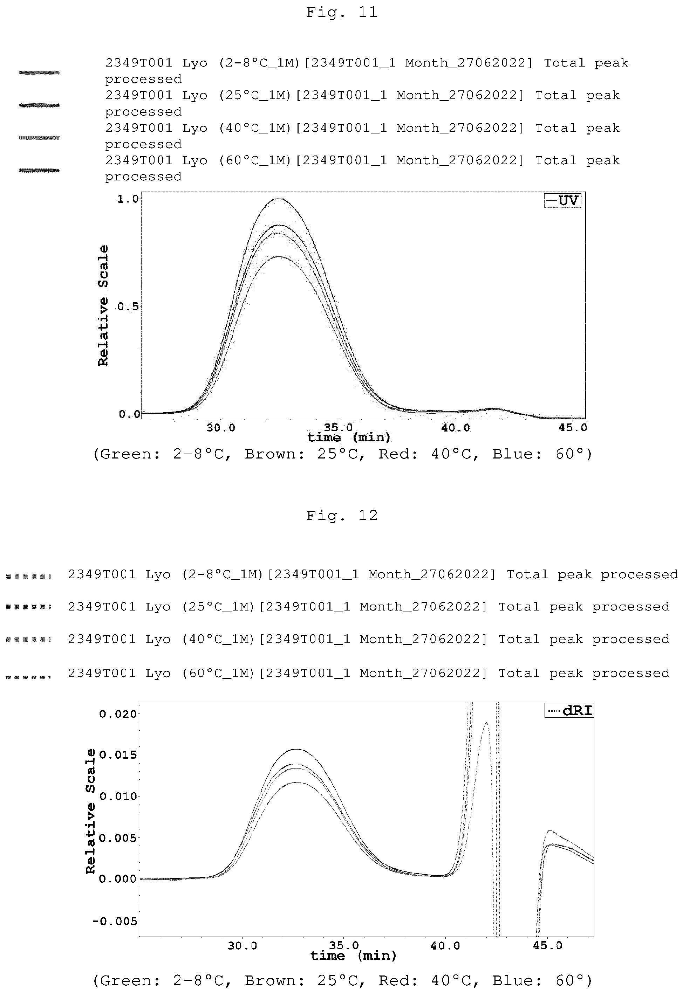

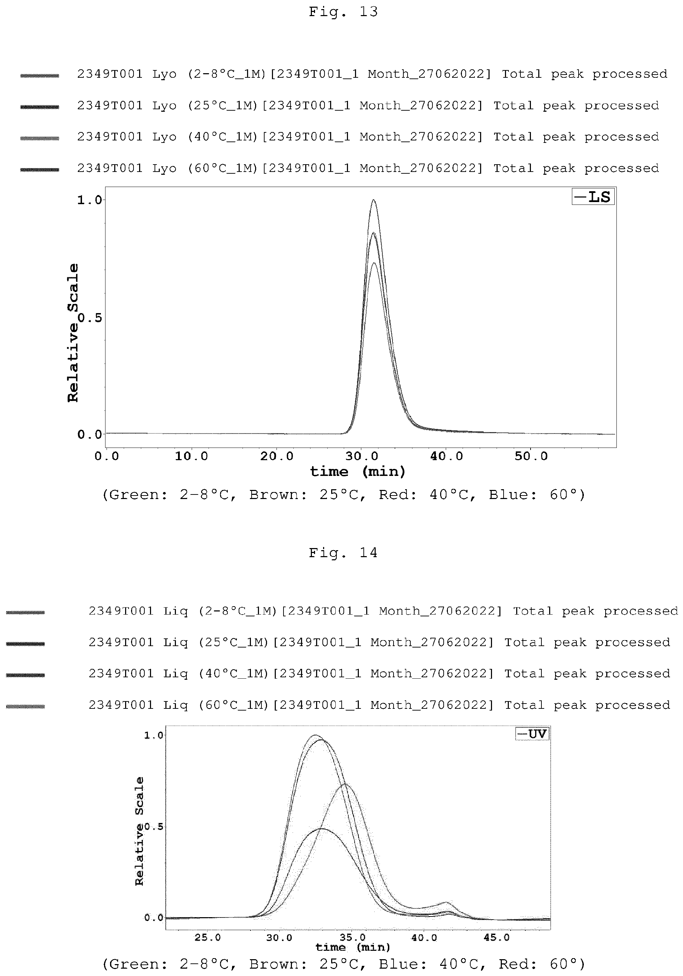

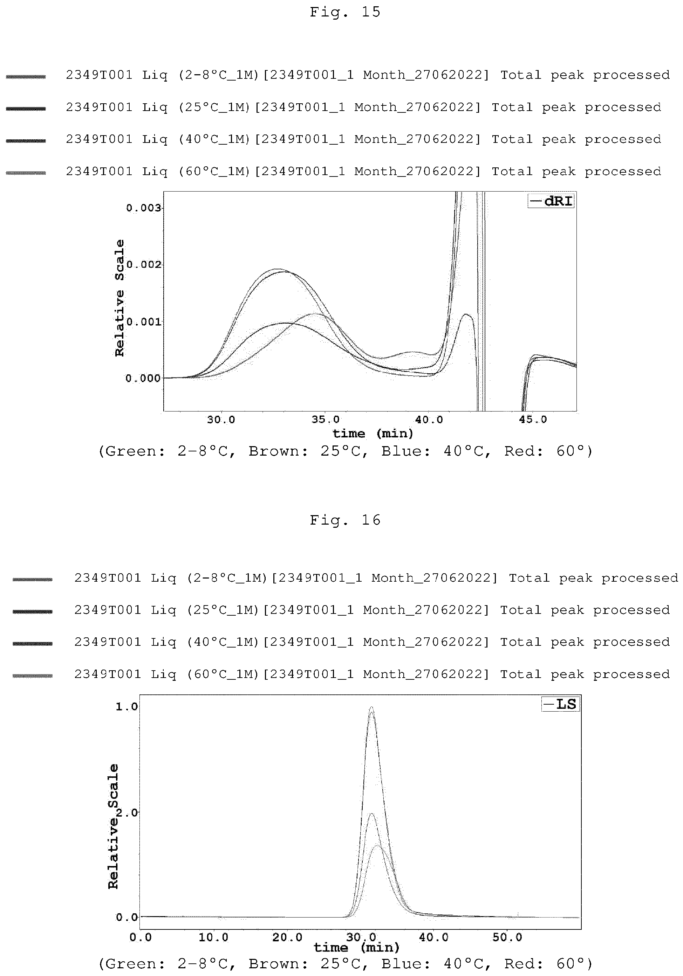

From Table 14 and Figure 3 to 6 and 11 to 16; following observations were found.

For HMW molecules: After one-month analysis,

- For Lyophilized vaccine; when compared to control readings at 2-8°C, at temperature condition 25°C, presence of HMW molecules decreases which is indicated by decrease in weight (Kilodalton) of the molecules.
- For Liquid vaccine; when compared to control readings at 2-8°C, at temperature condition 25°C, presence of HMW molecules increases which is indicated by increase in weight (Kilodalton) of the molecules.
- For Lyophilized vaccine; when compared to control readings at 2-8°C, at temperature condition 40°C, the HMW molecules increases.
- For Liquid vaccine; at temperature condition 40°C, there is no change in the presence of HMW molecules when compared with the initial (2-8°C).
- For Lyophilized vaccine; when compared to control readings at 2-8°C, at temperature condition 60°C, the HMW molecules decreases up to 12790.
- For Liquid vaccine; when compared to control readings at 2-8°C, at temperature condition 60°C, the HMW molecules increases in large quantity.

For LMW molecules: After one-month analysis,

- For Lyophilized vaccine; there are no major changes in the presence of LMW molecules at all temperature conditions 25°C, 40°C and 60°C as compared to the initial (2-8°C) temperature condition.
- For Liquid vaccine; when compared to control readings at 2-8°C, at temperature condition 25°C, presence of LMW molecules decreases which is indicated by decrease in weight (Kilodalton) of the molecules.
- For Liquid vaccine; when compared to control readings at 2-8°C, at temperature condition 40°C, the LMW molecules decreases.

- For Liquid vaccine; when compared to control readings at 2-8°C, at temperature condition 60°C, the LMW molecules decreases up to 598.

For AMW molecules: After one-month analysis,

- For Lyophilized vaccine; there are no major changes in the presence of AMW molecules at all temperature conditions 25°C, 40°C and 60°C as compared to the initial (2-8°C) temperature condition.
- For Liquid vaccine; when compared with the initial AMW molecules i.e. at 2-8°C, there is no major change in the presence on AMW molecules at 25°C.
- For Liquid vaccine; when compared with the initial AMW molecules i.e. at 2-8°C, the presence of AMW molecules is found to be decreased at 40°C and 60°C respectively.

**Conclusion:**

**For Lyophilized vaccine:**

- Presence of HMW molecules indicate the occurrence of aggregation. As compared to the initial/control readings in the above table, decrease in molecular weight of HMW molecules indicates aggregation is reduced at one month, 25°C; at one month, 40°C increase in molecular weight of HMW molecules as compared to the initial/control readings indicates aggregation is increased; whereas at one month, 60°C decrease in the molecular weight of HMW molecules indicate decrease in aggregation.
- As compared to the initial/control readings in the above table, there is no change in the molecular weights (Kilodalton) of LMW molecules which indicate that there is no any change in the amt of free polysaccharide or protein molecules present in the vaccine sample.
- As compared to the initial/control readings in the above table, there is no change in the molecular weights (Kilodalton) of AMW molecules which indicate that there is no any change in the amt of free polysaccharide or protein molecules present in the vaccine sample.

**For Liquid vaccine:**

- Presence of HMW molecules indicate the occurrence of aggregation. As compared to the initial/control readings in the above table, increase in molecular weight of HMW molecules indicates aggregation is increased at one month, 25°C; at one month, 40°C when compared to the initial/control readings no change in the presence of HMW molecules indicates there is no any change in the aggregation / aggregate molecules; whereas at one month, 60°C increase in the molecular weight of HMW molecules indicate increase in aggregation.
- When compared to the initial/control readings in the above table, the decrease in the molecular weights (Kilodalton) of LMW molecules indicates that the amount of free polysaccharide or protein molecules present in the vaccine sample is reduced at specific temperature conditions 25°C, 40°C, 60°C respectively.
- At 25°C, when compared to the initial/control readings in the above table, the no change in the molecular weights of AMW molecules, indicates no change in the presence of AMW molecules. When compared to the initial/control readings in the above table, the decrease in the molecular weights (Kilodalton) of AMW molecules indicates that the amount of free polysaccharide or protein molecules present in the vaccine sample is reduced at specific temperature conditions 40°C, and 60°C respectively.

Table 15: Comparison between Lyophilized and Liquid vaccine of 5 Dose at 2-8°C (initial), 25°C, 40°C, 60°C respectively

| | 5 Dose | | | |
| | Comparison between Lyophilized Vs Liquid Vaccine | | | |
| Temperature | HMW | Main Peak | LMW | AMW |
| 2-8°C (Lyo) | 16250 | 5826 | 2822 | 5767 |

(continued)

| | 5 Dose | | | |
|---|---|---|---|---|
| | Comparison between Lyophilized Vs Liquid Vaccine | | | |
| Temperature | HMW | Main Peak | LMW | AMW |
| 2-8°C (Liq) | 12880 | 5885 | 2848 | 5744 |
| Temperature | HMW | Main Peak | LMW | AMW |
| 25°C (Lyo) | 14720 | 6052 | 3184 | 6093 |
| 25°C (Liq) | 14690 | 5787 | 2093 | 5567 |
| Temperature | HMW | Main Peak | LMW | AMW |
| 40°C (Lyo) | 14980 | 6067 | 3156 | 6015 |
| 40°C (Liq) | 15530 | 5558 | 1145 | 5061 |
| Temperature | HMW | Main Peak | LMW | AMW |
| 60°C (Lyo) | 11800 | 5762 | 2419 | 5665 |
| 60°C (Liq) | 18120 | 4268 | 577 | 3234 |

**Observations:**

Table 15 focuses on the presentation of molecules in terms of molecular weight.

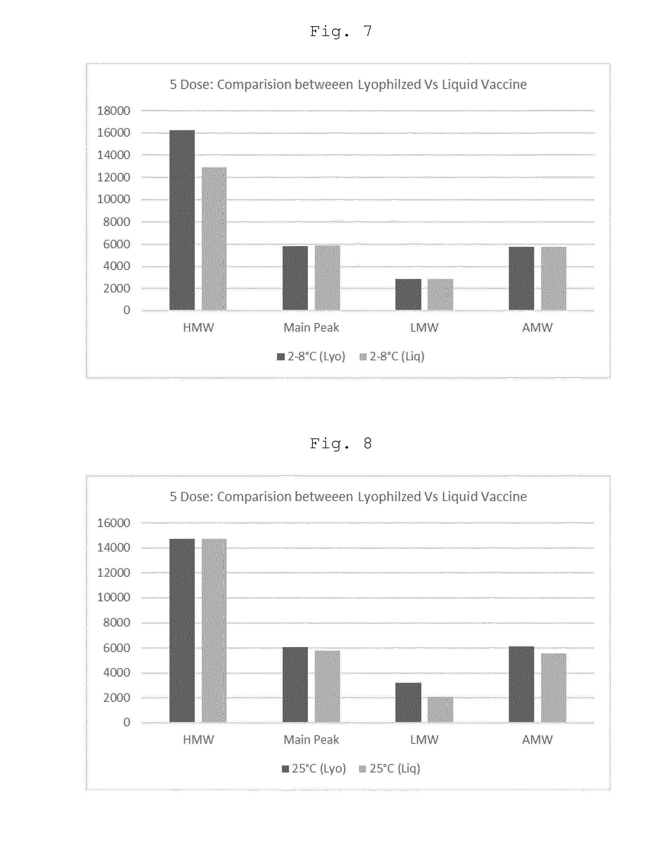

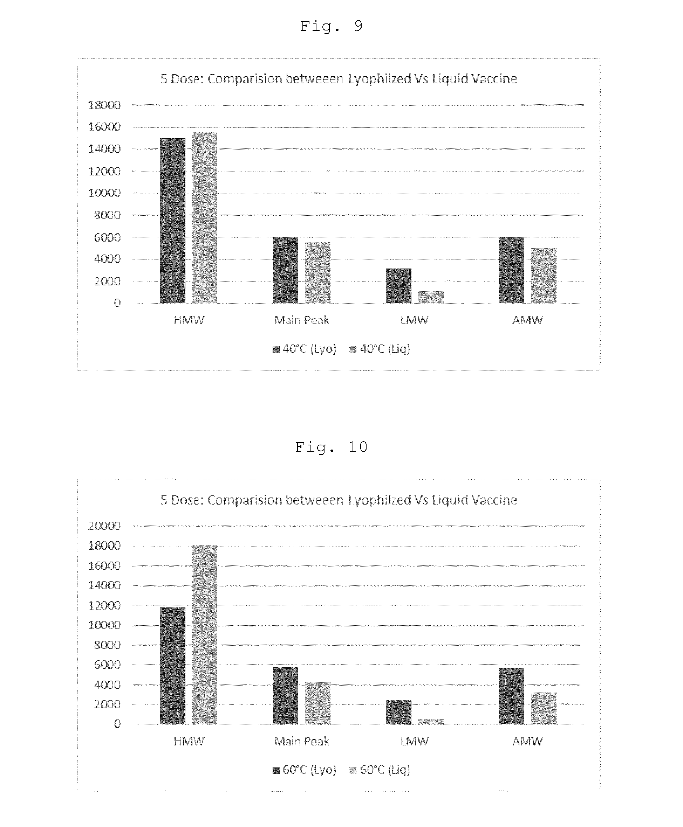

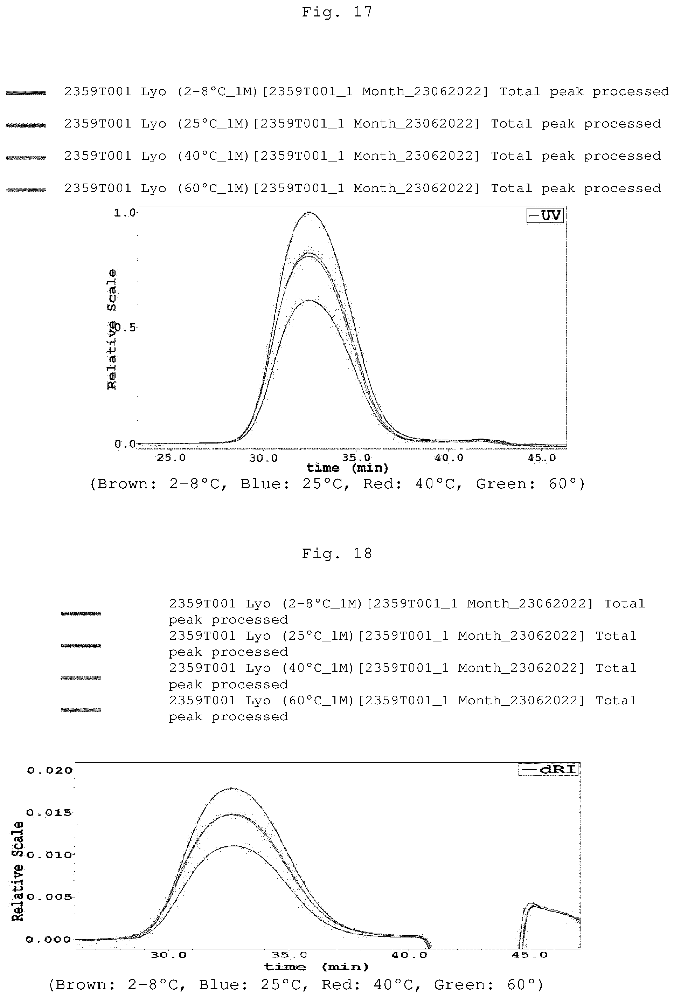

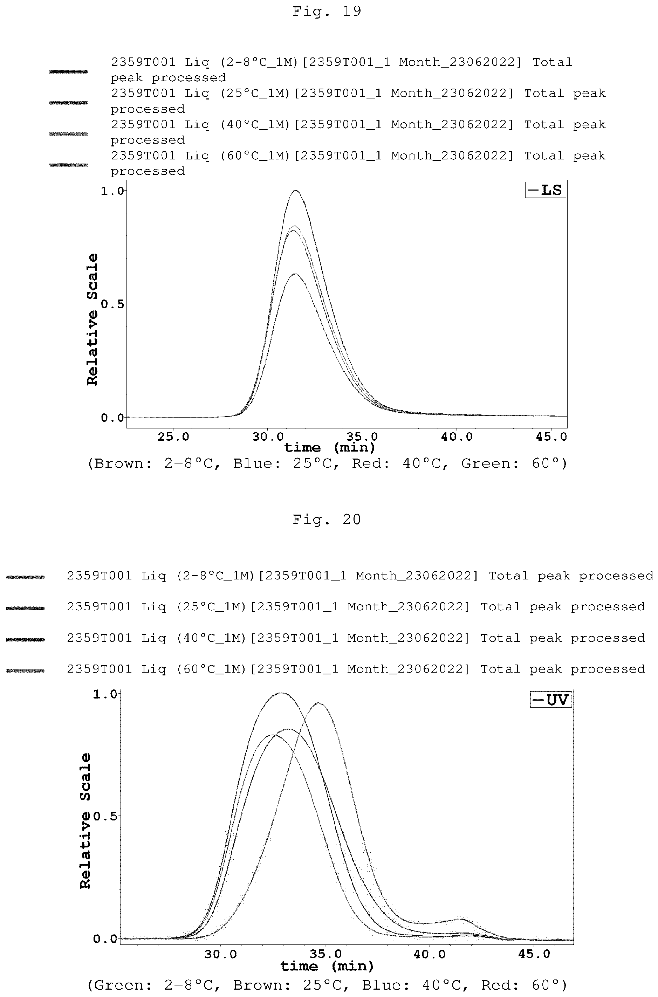

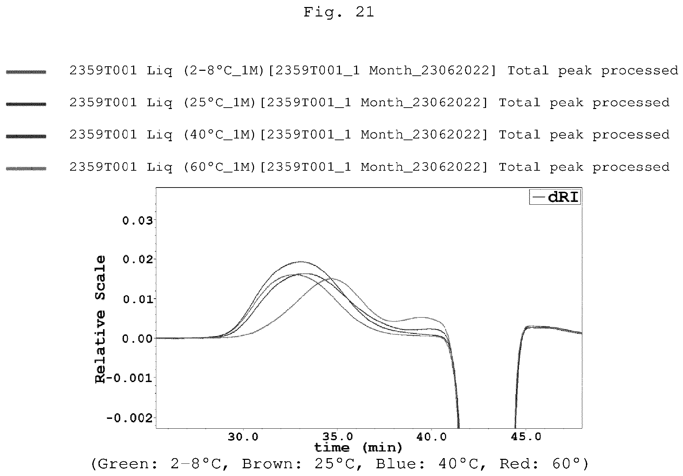

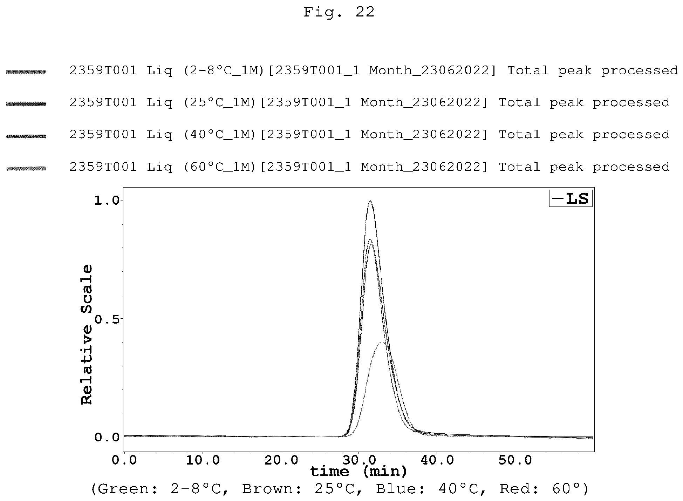

From Table 15 and Figure 7 to 10 and 17 to 22; following observations were found.

For HMW molecules: After one-month analysis,

- For Lyophilized vaccine; when compared to control readings at 2-8°C, at temperature condition 25°C, presence of HMW molecules decreases which is indicated by decrease in weight (Kilodalton) of the molecules.
- For Liquid vaccine; when compared to control readings at 2-8°C, at temperature condition 25°C, presence of HMW molecules increases which is indicated by increase in weight (Kilodalton) of the molecules.
- For Lyophilized vaccine; when compared to control readings at 2-8°C, at temperature condition 40°C, the HMW molecules decreases.
- For Liquid vaccine; at temperature condition 40°C, the HMW molecules decreases when compared with the initial (2-8°C).
- For Lyophilized vaccine; when compared to control readings at 2-8°C, at temperature condition 60°C, the HMW molecules decreases in the large quantity up to 11800.
- For Liquid vaccine; when compared to control readings at 2-8°C, at temperature condition 60°C, the HMW molecules increases in large quantity.

For LMW molecules: After one-month analysis,

- For Lyophilized vaccine; there are no major changes in the presence of LMW molecules at all temperature conditions 25°C, 40°C and 60°C as compared to the initial (2-8°C) temperature condition.
- For Liquid vaccine; when compared to control readings at 2-8°C, at temperature condition 25°C, presence of LMW molecules decreases which is indicated by decrease in weight (Kilodalton) of the molecules.
- For Liquid vaccine; when compared to control readings at 2-8°C, at temperature condition 40°C, the LMW molecules decreases.
- For Liquid vaccine; when compared to control readings at 2-8°C, at temperature condition 60°C, the LMW molecules decreases up to 577.

For AMW molecules: After one-month analysis,

- For Lyophilized vaccine; there are no major changes in the presence of AMW molecules at all temperature conditions 25°C, 40°C and 60°C as compared to the initial (2-8°C) temperature condition.
- For Liquid vaccine; when compared with the initial AMW molecules i.e. at 2-8°C, there is no major change in the presence of AMW molecules at 25°C.
- For Liquid vaccine; when compared with the initial AMW molecules i.e. at 2-8°C, the presence of AMW molecules is found to be decreased at 40°C and decreased in large quantity at 60°C respectively.

**Conclusion:**

**For Lyophilized vaccine:**

- Presence of HMW molecules indicate the occurrence of aggregation. As compared to the initial/control readings in the above table, decrease in molecular weight of HMW molecules indicates aggregation is reduced at one month, 25°C; at one month, 40°C decrease in molecular weight of HMW molecules as compared to the initial/control readings indicates aggregation is reduced; whereas at one month, 60°C decrease in the molecular weight of HMW molecules indicate decrease in aggregation.
- As compared to the initial/control readings in the above table, there is no change in the molecular weights (Kilodalton) of LMW molecules which indicate that there is no any change in the amt of free polysaccharide or protein molecules present in the vaccine sample.
- As compared to the initial/control readings in the above table, there is no change in the molecular weights (Kilodalton) of AMW molecules which indicate that there is no any change in the amt of free polysaccharide or protein molecules present in the vaccine sample.

**For Liquid vaccine:**

- Presence of HMW molecules indicate the occurrence of aggregation. As compared to the initial/control readings in the above table, increase in molecular weight of HMW molecules indicates aggregation is increased at one month, 25°C; at one month, 40°C when compared to the initial/control readings the decrease in the presence of HMW molecules indicates there is reduction in the aggregation / aggregate molecules; whereas at one month, 60°C increase in the molecular weight of HMW molecules in large quantity indicate increase in aggregation.
- When compared to the initial/control readings in the above table, the decrease in the molecular weights (Kilodalton) of LMW molecules indicates that the amount of free polysaccharide or protein molecules present in the vaccine sample is reduced at specific temperature conditions 25°C, 40°C, 60°C respectively.
- At 25°C, when compared to the initial/control readings in the above table, the no change in the molecular weights of AMW molecules, indicates no change in the presence of AMW molecules. When compared to the initial/control readings in the above table, the decrease in the molecular weights (Kilodalton) of AMW molecules indicates that the amount of free polysaccharide or protein molecules present in the vaccine sample is reduced at specific temperature conditions 40°C, and 60°C respectively.

**Claims**

1. A method for determining the stability of a polysaccharide-protein conjugate vaccine, said method comprising the following steps:

   a) subjecting the said polysaccharide-protein conjugate vaccine to a high performance size exclusion chromatography (HPLC-SEC) using a mobile phase and set of three chromatography columns in series to obtain an eluate;
   b) passing the said eluate through detectors and evaluating the eluate to obtain the distribution of the molecules with respect to molecular weight; and
   c) analysing the molecular weight to obtain molecular size and/or molar mass profile of the polysaccharide-protein conjugate vaccine based on the percentage of high molecular weight (HMW), average molecular weight (AMW) and low molecular weight (LMW) molecules,

   wherein the high molecular weight is in the range from 13000 kDa to 19000 kDa, average molecular weight is in the range of 2000 kDa to 11000 kDa and low molecular weight is in the range of 200 kDa to 2000 kDa for lyophilized polysaccharide-protein conjugate vaccine, or
   wherein the high molecular weight is in the range from 10000 kDa to 19000 kDa, average molecular weight is in the range of 1000 kDa to 10000 kDa and low molecular weight is in the range of 200 kDa to 1000 kDa for liquid polysaccharide-protein conjugate vaccine;

d) correlating the range (%) of High molecular weight (HMW) and Low molecular weight (LMW) with stability, immunogenicity and potency/efficacy of the vaccine composition or the ratio of average molecular weight (AMW), high molecular weight (HMW) and Low molecular weight (LMW) with stability, immunogenicity and potency/efficacy of the vaccine composition.

2. The method as claimed in claim 1, wherein a pre-treatment step is carried out before step a).

3. The method as claimed in claim 2, wherein the pre-treatment step comprises of reconstitution of polysaccharide-protein conjugate vaccine using a buffer.

4. The method as claimed in claim 3, wherein the buffer used for reconstitution of polysaccharide-protein conjugate vaccine in pre-treatment step is selected from phosphate buffer saline, Tris, MES, HEPES, citrate and combinations thereof.

5. The method as claimed in claim 4, wherein the buffer used for reconstitution of polysaccharide-protein conjugate vaccine in pre-treatment step is Tris buffer.

6. The method as claimed in claim 1, wherein the set of three chromatography columns in series is connected with a guard column.

7. The method as claimed in claim 6, wherein the arrangement of columns is guard column followed by first column, first column followed by second column and second column followed by third column.

8. The method as claimed in claim 6, wherein the guard column comprises polymer based packed material having particle size 9 - 14 $\mu$m.

9. The method as claimed in claim 8, wherein the polymer based packed material of guard column comprises polyhydroxymethacrylate based material.

10. The method as claimed in claim 7, wherein the first and second chromatography columns comprises polyhydroxymethacrylate based material and third chromatography column is selected from hydroxylated polymethacrylate based material and hydrophilic vinyl polymer based material.

11. The method as claimed in claims 7 and 10, wherein particle size of first column is 34 - 36 $\mu$m, particle size of second column is 12 - 14 $\mu$m and particle size of third column is 9 - 14 $\mu$m.

12. The method as claimed in claim 1, wherein the length of the set of three chromatography columns in series ranges from 85 - 95 cm.

13. The method as claimed in claim 6, wherein the guard column and chromatography columns in series are connected using a connector.

14. The method as claimed in claim 1, wherein the detector in step b) is selected from UV detector, refractive index (RI) detector and multiangle light scattering (MALS) detector and combinations thereof.

15. The method as claimed in claim 1, wherein the mobile phase is a buffer selected from phosphate buffer saline, Tris, MES, HEPES, citrate and combinations thereof.

16. The method as claimed in claim 15, wherein the buffer is phosphate buffer saline having pH ranging from 7.2 to 7.5, preferably pH 7.4.

17. The method as claimed in claim 16, wherein the phosphate buffer saline comprises of sodium chloride in the range of 20 to 40 g, preferably 23.38 g.

18. The method as claimed in claim 1, wherein the high performance size exclusion chromatography (HPLC-SEC) in step a) comprises a flow rate ranging from 0.1 to 1 ml per minute, preferably 0.30 to 0.80 ml per minute.

19. The method as claimed in claim 1, wherein the high performance size exclusion chromatography (HPLC-SEC) in step a) comprises a column temperature ranging from 25°C to 35°C.

20. The method as claimed in claim 1, wherein the high performance size exclusion chromatography (HPLC-SEC) in step a) comprises injection run time ranging from 60 - 70 min.

21. The method as claimed in claim 1, wherein the polysaccharide-protein conjugate vaccine is in liquid form or lyophilized form.

22. The method as claimed in claim 1, wherein the polysaccharide is a bacterial capsular polysaccharide, and is obtained from group comprising of Helicobacter pylori, Chlamydia pneumoniae, Chlamydia trachomatis, Ureaplasma urealyticum, Mycoplasma pneumoniae, Staphylococcus spp., Staphylococcus aureus, Streptococcus spp., Group A Streptococcus, Group B Streptococcus, Streptococcus agalactiae, Streptococcus pyogenes, Streptococcus pneumoniae, Streptococcus viridans, Enterococcus faecalis, Neisseria meningitidis, Neisseria gonorrhoeae, Bacillus anthracis, Salmonella spp., Salmonella typhi, Salmonella paratyphi, Salmonella enteritidis, Salmonella typhimurium, Vibrio cholerae, Pasteurella pestis, Pseudomonas aeruginosa, Campylobacter spp., Campylobacter jejuni, Clostridium spp., Clostridium difficile, Mycobacterium spp., Mycobacterium tuberculosis, Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Hemophilus influenzae, Escherichia coli, Shigella spp., Ehrlichia spp., and Rickettsia spp..

23. The method as claimed in claim 22, wherein polysaccharide is obtained from one or more of the following:

   a. Streptococcus pneumoniae serotype 1, 2, 3, 4, 5, 6, 6A, 6B, 6C, 6D, 6E, 6G, 6H, 7A, 7B, 7C, 7F, 8, 9A, 9L, 9F, 9N, 9V, 10F, 10B, 10C, 10A, 11 A, 11F, 11B, 11C, 11D, 11E, 12A, 12B, 12F, 13, 14, 15A, 15C, 15B, 15F,16A, 16F, 17A, 17F, 18, 18C, 18F, 18A, 18B, 19A, 19B, 19C, 19F, 20, 20A, 20B, 21, 22A, 22F, 23A, 23B, 23F, 24A, 24B, 24F, 25F, 25A, 27, 28F, 28A, 29, 31, 32F, 32A, 33A, 33C, 33D, 33E, 33F, 33B, 34, 45, 38, 35A, 35B, 35C, 35F, 36, 37, 38, 39, 40, 41F, 41A, 42, 43, 44, 45, 46, 47F, 47A, 48;
   b. Neisseria meningitidis serotypes A, B, C, D, W135, X, Y, Z, and 29E;
   c. Haemophilus influenzae type b;
   d. Salmonella spp. including salmonella typhi, salmonella paratyphi A, salmonella paratyphi B, salmonella paratyphi C, salmonella typhimurium, and salmonella enteritidis;
   e. Streptococcus spp. including Group A Streptococcus and Group B Streptococcus, Streptococcus group (Ia, Ib, II, III, IV, V, VI, VII, VIII, and IX).

24. The method as claimed in claim 1, wherein the polysaccharide-protein conjugate vaccine to be evaluated comprises carrier protein selected from the group of CRM197, diphtheria toxin/toxoid, Neisseria meningitidis outer membrane complex, fragment C of tetanus toxoid, tetanus toxin/toxoid, pertussis toxin/toxoid, flagellin (FliC), cholera toxin B subunit (CTB), protein D of H. influenzae, E. coli LT, E. coli ST, and exotoxin A from Pseudomonas aeruginosa, outer membrane complex c (OMPC), porins (Por A, Por B), transferrin binding proteins, pneumolysin, pneumococcal surface protein A (PspA), pneumococcal surface adhesin A (PsaA), PhtA, PhtB, PhtE, pneumococcal PhtD, pneumococcal surface proteins BVH-3 and BVH-11, M. catarrhalis uspA, protective antigen (PA) of Bacillus anthracis and detoxified edema factor (EF) and lethal factor (LF) of Bacillus anthracis, ovalbumin, keyhole limpet hemocyanin (KLH), C5a peptidase group A or group B Streptococcus, human serum albumin, bovine serum albumin (BSA), NTHi high molecular weight protein, fHbp, purified protein derivative of tuberculin (PPD) and Cholera toxin subunit B.

25. The method as claimed in claim 23, wherein the polysaccharide is selected from Neisseria meningitidis serotypes A, C, Y, W135 and X.

26. The method as claimed in claim 24, wherein the carrier protein is selected from CRM197, tetanus toxoid (TT) and combinations thereof.

27. The method as claimed in claim 1, wherein the polysaccharide-protein conjugate vaccine is a multivalent vaccine.

28. The method as claimed in claim 1, wherein the polysaccharide-protein conjugate vaccine is a monovalent vaccine.

29. The method as claimed in claim 27, wherein the multivalent vaccine comprises of Neisseria meningitidis serotype A - TT conjugate, Neisseria meningitidis serotype X - TT conjugate, Neisseria meningitidis serotype C - CRM197 conjugate, Neisseria meningitidis serotype Y - CRM197 conjugate and Neisseria meningitidis serotype W - CRM197 conjugate.

**Patentansprüche**

1. Verfahren zur Bestimmung der Stabilität eines Polysaccharid-Protein-Konjugat-Impfstoffs, wobei das Verfahren die folgenden Schritte umfasst:

   a) Unterziehen des Polysaccharid-Protein-Konjugat-Impfstoffs einer Hochleistungsgrößenausschlusschromatographie (HPLC-SEC) unter Verwendung einer mobilen Phase und eines Satzes von drei Chromatographiesäulen in Reihe, um ein Eluat zu erhalten;
   b) Leiten des Eluats durch Detektoren und Auswerten des Eluats, um die Verteilung der Moleküle in Bezug auf das Molekulargewicht zu erhalten; und
   c) Analysieren des Molekulargewichts, um ein Molekülgrößen- und/oder Molmassenprofil des Polysaccharid-Protein-Konjugat-Impfstoffs auf der Grundlage des prozentualen Anteils an Molekülen mit hohem Molekulargewicht (HMW), mittlerem Molekulargewicht (AMW) und niedrigem Molekulargewicht (LMW) zu erhalten,

   wobei das hohe Molekulargewicht im Bereich von 13000 kDa bis 19000 kDa, das mittlere Molekulargewicht im Bereich von 2000 kDa bis 11000 kDa und das niedrige Molekulargewicht im Bereich von 200 kDa bis 2000 kDa für lyophilisierten Polysaccharid-Protein-Konjugat-Impfstoff liegt, oder
   wobei das hohe Molekulargewicht im Bereich von 10000 kDa bis 19000 kDa, das mittlere Molekulargewicht im Bereich von 1000 kDa bis 10000 kDa und das niedrige Molekulargewicht im Bereich von 200 kDa bis 1000 kDa für flüssigen Polysaccharid-Protein-Konjugat-Impfstoff liegt;

   d) Korrelieren des Bereichs (%) von Hochmolekulargewicht (HMW) und Niedermolekulargewicht (LMW) mit Stabilität, Immunogenität und Potenz/Wirksamkeit der Impfstoffzusammensetzung oder des Verhältnisses von mittlerem Molekulargewicht (AMW), Hochmolekulargewicht (HMW) und Niedermolekulargewicht (LMW) mit Stabilität, Immunogenität und Potenz/Wirksamkeit der Impfstoffzusammensetzung.

2. Verfahren nach Anspruch 1, wobei vor Schritt a) ein Vorbehandlungsschritt durchgeführt wird.

3. Verfahren nach Anspruch 2, wobei der Vorbehandlungsschritt die Rekonstitution des Polysaccharid-Protein-Konjugat-Impfstoffs unter Verwendung eines Puffers umfasst.

4. Verfahren nach Anspruch 3, wobei der Puffer, der zur Rekonstitution des Polysaccharid-Protein-Konjugat-Impfstoffs im Vorbehandlungsschritt verwendet wird, aus Phosphatpuffersalzlösung, Tris, MES, HEPES, Citrat und Kombinationen davon ausgewählt ist.

5. Verfahren nach Anspruch 4, wobei der Puffer, der zur Rekonstitution des Polysaccharid-Protein-Konjugat-Impfstoffs im Vorbehandlungsschritt verwendet wird, Tris-Puffer ist.

6. Verfahren nach Anspruch 1, wobei der Satz von drei Chromatographiesäulen in Reihe mit einer Vorsäule verbunden ist.

7. Verfahren nach Anspruch 6, wobei die Anordnung von Säulen eine Vorsäule, gefolgt von einer ersten Säule, eine erste Säule, gefolgt von einer zweiten Säule und eine zweite Säule, gefolgt von einer dritten Säule, ist.

8. Verfahren nach Anspruch 6, wobei die Vorsäule gepacktes Material auf Polymerbasis mit einer Partikelgröße von 9 - 14 $\mu$m umfasst.

9. Verfahren nach Anspruch 8, wobei das gepackte Material auf Polymerbasis der Vorsäule Material auf Polyhydroxymethacrylatbasis umfasst.

10. Verfahren nach Anspruch 7, wobei die erste und zweite Chromatographiesäule Material auf Polyhydroxymethacrylatbasis umfasst und die dritte Chromatographiesäule aus hydroxyliertem Material auf Polymethacrylatbasis und hydrophilem Material auf Vinylpolymerbasis ausgewählt ist.

11. Verfahren nach den Ansprüchen 7 und 10, wobei die Partikelgröße der ersten Säule 34 - 36 μm beträgt, die Partikelgröße der zweiten Säule 12 - 14 μm beträgt und die Partikelgröße der dritten Säule 9 - 14 μm beträgt.

12. Verfahren nach Anspruch 1, wobei die Länge des Satzes von drei Chromatographiesäulen in Reihe im Bereich von 85 bis 95 cm liegt.

13. Verfahren nach Anspruch 6, wobei die Vorsäule und die Chromatographiesäulen in Reihe unter Verwendung eines Verbinders verbunden sind.

14. Verfahren nach Anspruch 1, wobei der Detektor in Schritt b) ausgewählt ist aus einem UV-Detektor, einem Brechungsindex (RI)-Detektor und einem Mehrwinkel-Lichtstreuung (MALS)-Detektor und Kombinationen davon.

15. Verfahren nach Anspruch 1, wobei die mobile Phase ein Puffer ist, der aus Phosphatpuffersalzlösung, Tris, MES, HEPES, Citrat und Kombinationen davon ausgewählt ist.

16. Verfahren nach Anspruch 15, wobei der Puffer eine Phosphatpuffersalzlösung mit einem pH-Wert im Bereich von 7,2 bis 7,5, vorzugsweise pH 7,4, ist.

17. Verfahren nach Anspruch 16, wobei die Phosphatpuffersalzlösung Natriumchlorid im Bereich von 20 bis 40 g, vorzugsweise 23,38 g, umfasst.

18. Verfahren nach Anspruch 1, wobei die Hochleistungsgrößenausschlusschromatographie (HPLC-SEC) in Schritt a) eine Flussrate im Bereich von 0,1 bis 1 ml pro Minute, vorzugsweise 0,30 bis 0,80 ml pro Minute umfasst.

19. Verfahren nach Anspruch 1, wobei die Hochleistungsgrößenausschlusschromatographie (HPLC-SEC) in Schritt a) eine Säulentemperatur im Bereich von 25 °C bis 35 °C umfasst.

20. Verfahren nach Anspruch 1, wobei die Hochleistungsgrößenausschlusschromatographie (HPLC-SEC) in Schritt a) eine Injektionslaufzeit im Bereich von 60 - 70 min umfasst.

21. Verfahren nach Anspruch 1, wobei der Polysaccharid-Protein-Konjugat-Impfstoff in flüssiger Form oder lyophilisierter Form vorliegt.

22. Verfahren nach Anspruch 1, wobei das Polysaccharid ein bakterielles kapselförmiges Polysaccharid ist und aus einer Gruppe erhalten wird, die Helicobacter pylori, Chlamydia pneumoniae, Chlamydia trachomatis, Ureaplasma urealyticum, Mycoplasma pneumoniae, Staphylococcus spp, Staphylococcus aureus, Streptococcus spp., Streptokokken der Gruppe A, Streptokokken der Gruppe B, Streptococcus agalactiae, Streptococcus pyogenes, Streptococcus pneumoniae, Streptococcus viridans, Enterococcus faecalis, Neisseria meningitidis, Neisseria gonorrhoeae, Bacillus anthracis, Salmonella spp, Salmonella typhi, Salmonella paratyphi, Salmonella enteritidis, Salmonella typhimurium, Vibrio cholerae, Pasteurella pestis, Pseudomonas aeruginosa, Campylobacter spp, Campylobacter jejuni, Clostridium spp, Clostridium difficile, Mycobacterium spp., Mycobacterium tuberculosis, Treponema spp, Borrelia spp., Borrelia burgdorferi, Leptospira spp., Hemophilus ducreyi, Corynebacterium diphtheria, Bordetella pertussis, Bordetella parapertussis, Bordetella bronchiseptica, Hemophilus influenzae, Escherichia coli, Shigella spp., Ehrlichia spp., und Rickettsia spp. umfasst.

23. Verfahren nach Anspruch 22, wobei das Polysaccharid aus einem oder mehreren der folgenden gewonnen wird:

    a. Streptococcus pneumoniae Serotyp 1, 2, 3, 4, 5, 6, 6A, 6B, 6C, 6D, 6E, 6G, 6H, 7A, 7B, 7C, 7F, 8, 9A, 9L, 9F, 9N, 9V, 10F, 10B, 10C, 10A, 11A, 11F, 11B, 11C, 11D, 11E, 12A, 12B, 12F, 13, 14, 15A, 15C, 15B, 15F, 16A, 16F, 17A, 17F, 18, 18C, 18F, 18A, 18B, 19A, 19B, 19C, 19F, 20, 20A, 20B, 21, 22A, 22F, 23A, 23B, 23F, 24A, 24B, 24F, 25F, 25A, 27, 28F, 28A, 29, 31, 32F, 32A, 33A, 33C, 33D, 33E, 33F, 33B, 34, 45, 38, 35A, 35B, 35C, 35F, 36, 37, 38, 39, 40, 41F, 41A, 42, 43, 44, 45, 46, 47F, 47A, 48;
    b. Neisseria meningitidis Serotypen A, B, C, D, W135, X, Y, Z, und 29E;
    c. Haemophilus influenzae Typ b;
    d. Salmonella spp. einschließlich Salmonella typhi, Salmonella paratyphi A, Salmonella paratyphi B, Salmonella paratyphi C, Salmonella typhimurium und Salmonella enteritidis;
    e. Streptococcus spp. einschließlich Streptokokken der Gruppe A und der Gruppe B, Streptokokkengruppe (Ia, Ib, II, III, IV, V, VI, VII, VIII und IX).

24. Verfahren nach Anspruch 1, wobei der zu auszuwertende Polysaccharid-Protein-Konjugat-Impfstoff ein Trägerprotein umfasst, ausgewählt aus der Gruppe von CRM197, Diphtherietoxin/Toxoid, äußerer Membrankomplex von Neisseria meningitidis, Fragment C des Tetanustoxoids, Tetanustoxin/Toxoid, Keuchhusten-Toxin/Toxoid, Flagellin (FliC), Choleratoxin-B-Untereinheit (CTB), Protein D von H. influenzae, E. coli LT, E. coli ST und Exotoxin A von Pseudomonas aeruginosa, äußerer Membrankomplex C (OMPC), Porine (Por A, Por B), Transferrin-bindende Proteine, Pneumolysin, Pneumokokken-Oberflächenprotein A (PspA), Pneumokokken-Oberflächenadhäsin A (PsaA), PhtA, PhtB, PhtE, Pneumokokken-PhtD, Pneumokokken-Oberflächenproteine BVH-3 und BVH-11, M. catarrhalis uspA, protektivem Antigen (PA) von Bacillus anthracis und entoxifiziertem Ödemfaktor (EF) und Letalfaktor (LF) von Bacillus anthracis, Ovalbumin, Keyhole-Limpet-Hämocyanin (KLH), C5a-Peptidase von Streptokokken der Gruppe A oder B, humanem Serumalbumin, Rinderserumalbumin (BSA), NTHi-Protein mit hohem Molekulargewicht, fHbp, gereinigtem Tuberkulin-Derivat (PPD) und Choleratoxin-Untereinheit B.

25. Verfahren nach Anspruch 23, wobei das Polysaccharid aus den Serotypen A, C, Y, W135 und X von Neisseria meningitidis ausgewählt ist.

26. Verfahren nach Anspruch 24, wobei das Trägerprotein aus CRM197, Tetanustoxoid (TT) und Kombinationen davon ausgewählt ist.

27. Verfahren nach Anspruch 1, wobei der Polysaccharid-Protein-Konjugat-Impfstoff ein multivalenter Impfstoff ist.

28. Verfahren nach Anspruch 1, wobei der Polysaccharid-Protein-Konjugat-Impfstoff ein monovalenter Impfstoff ist.

29. Verfahren nach Anspruch 27, wobei der multivalente Impfstoff Neisseria meningitidis Serotyp A - TT-Konjugat, Neisseria meningitidis Serotyp X - TT-Konjugat, Neisseria meningitidis Serotyp C - CRM197-Konjugat, Neisseria meningitidis Serotyp Y - CRM197-Konjugat und Neisseria meningitidis Serotyp W - CRM197-Konjugat umfasst.

**Revendications**

1. Procédé de détermination de la stabilité d'un vaccin conjugué polysaccharide-protéine, ledit procédé comprenant les étapes suivantes :

   a) soumettre ledit vaccin conjugué polysaccharide-protéine à une chromatographie d'exclusion de taille à haute performance (HPLC-SEC) utilisant une phase mobile et un ensemble de trois colonnes de chromatographie en série pour obtenir un éluat ;
   b) faire passer ledit éluat à travers des détecteurs et évaluer l'éluat pour obtenir la distribution des molécules par rapport au poids moléculaire ; et
   c) analyser le poids moléculaire pour obtenir le profil de taille moléculaire et/ou de masse molaire du vaccin conjugué polysaccharide-protéine en fonction du pourcentage de molécules de poids moléculaire élevé (HMW), de poids moléculaire moyen (AMW) et de faible poids moléculaire (LMW),

   dans lequel le poids moléculaire élevé est compris entre 13 000 kDa et 19 000 kDa, le poids moléculaire moyen est compris entre 2 000 kDa et 11 000 kDa et le poids moléculaire faible est compris entre 200 kDa et 2 000 kDa pour le vaccin conjugué polysaccharide-protéine lyophilisé, ou
   dans lequel le poids moléculaire élevé est compris entre 10 000 kDa et 19 000 kDa, le poids moléculaire moyen est compris entre 1 000 kDa et 10 000 kDa et le poids moléculaire faible est compris entre 200 kDa et 1 000 kDa pour le vaccin liquide conjugué polysaccharide-protéine ;

   d) corréler la gamme (%) de poids moléculaire élevé (HMW) et de poids moléculaire faible (LMW) avec la stabilité, l'immunogénicité et la puissance/l'efficacité de la composition vaccinale ou le rapport entre le poids moléculaire moyen (AMW), le poids moléculaire élevé (HMW) et le poids moléculaire faible (LMW) avec la stabilité, l'immunogénicité et la puissance/l'efficacité de la composition vaccinale.

2. Procédé selon la revendication 1, dans lequel une étape de prétraitement est effectuée avant l'étape a).

3. Procédé selon la revendication 2, dans lequel l'étape de prétraitement comprend la reconstitution du vaccin conjugué polysaccharide-protéine à l'aide d'un tampon.

**4.** Procédé selon la revendication 3, dans lequel le tampon utilisé pour la reconstitution du vaccin conjugué polysaccharide-protéine dans l'étape de prétraitement est choisi parmi les tampons de solution saline de phosphate, Tris, MES, HEPES, citrate et leurs combinaisons.

**5.** Procédé selon la revendication 4, dans lequel le tampon utilisé pour la reconstitution du vaccin conjugué polysaccharide-protéine dans l'étape de prétraitement est un tampon Tris.

**6.** Procédé selon la revendication 1, dans lequel l'ensemble de trois colonnes de chromatographie en série est relié à une colonne de garde.

**7.** Procédé selon la revendication 6, dans lequel la disposition des colonnes est la suivante : colonne de garde suivie de la première colonne, première colonne suivie de la deuxième colonne et deuxième colonne suivie de la troisième colonne.

**8.** Procédé selon la revendication 6, dans lequel la colonne de garde comprend un matériau de remplissage à base de polymère dont la taille des particules est comprise entre 9 et 14 $\mu$m.

**9.** Procédé selon la revendication 8, dans lequel le matériau de remplissage à base de polymère de la colonne de garde comprend un matériau à base de polyhydroxyméthacrylate.

**10.** Procédé selon la revendication 7, dans lequel les première et deuxième colonnes de chromatographie comprennent un matériau à base de polyhydroxyméthacrylate et la troisième colonne de chromatographie est choisie parmi un matériau à base de polyméthacrylate hydroxylé et un matériau à base de polymère vinylique hydrophile.

**11.** Procédé selon les revendications 7 et 10, dans lequel la taille des particules de la première colonne est de 34 à 36 $\mu$m, la taille des particules de la deuxième colonne est de 12 à 14 $\mu$m et la taille des particules de la troisième colonne est de 9 à 14 $\mu$m.

**12.** Procédé selon la revendication 1, dans lequel la longueur du jeu de trois colonnes de chromatographie en série est comprise entre 85 et 95 cm.

**13.** Procédé selon la revendication 6, dans lequel la colonne de garde et les colonnes de chromatographie en série sont reliées à l'aide d'un connecteur.

**14.** Procédé selon la revendication 1, dans lequel le détecteur de l'étape b) est choisi parmi les détecteurs UV, les détecteurs d'indice de réfraction (RI) et les détecteurs de diffusion de la lumière sous plusieurs angles (MALS), ainsi que des combinaisons de ceux-ci.

**15.** Procédé selon la revendication 1, dans lequel la phase mobile est un tampon choisi parmi les tampons de solution saline de phosphate, Tris, MES, HEPES, citrate et leurs combinaisons.

**16.** Procédé selon la revendication 15, dans lequel le tampon est une solution tampon de phosphate saline ayant un pH compris entre 7,2 et 7,5, de préférence un pH de 7,4.

**17.** Procédé selon la revendication 16, dans lequel la solution tampon de phosphate saline comprend du chlorure de sodium compris entre 20 et 40 g, de préférence 23,38 g.

**18.** Procédé selon la revendication 1, dans lequel la chromatographie d'exclusion de taille à haute performance (HPLC-SEC) à l'étape a) comprend un débit allant de 0,1 à 1 ml par minute, de préférence de 0,30 à 0,80 ml par minute.

**19.** Procédé selon la revendication 1, dans lequel la chromatographie d'exclusion de taille à haute performance (HPLC-SEC) à l'étape a) comprend une température de colonne comprise entre 25°C et 35°C.

**20.** Procédé selon la revendication 1, dans lequel la chromatographie d'exclusion de taille à haute performance (HPLC-SEC) à l'étape a) comprend un temps d'injection compris entre 60 et 70 minutes.

**21.** Procédé selon la revendication 1, dans lequel le vaccin conjugué polysaccharide-protéine est sous forme liquide ou lyophilisée.

22. Procédé selon la revendication 1, dans lequel le polysaccharide est un polysaccharide capsulaire bactérien, et est obtenu à partir d'un groupe comprenant Helicobacter pylori, Chlamydia pneumoniae, Chlamydia trachomatis, Ureaplasma urealyticum, Mycoplasma pneumoniae, Staphylococcus spp, Staphylococcus aureus, Streptococcus spp, Streptocoques du groupe A, Streptocoques du groupe B, Streptococcus agalactiae, Streptococcus pyogenes, Streptococcus pneumoniae, Streptococcus viridans, Enterococcus faecalis, Neisseria meningitidis, Neisseria gonorrhoeae, Bacillus anthracis, Salmonella spp, Salmonella typhi, Salmonella paratyphi, Salmonella enteritidis, Salmonella typhimurium, Vibrio cholerae, Pasteurella pestis, Pseudomonas aeruginosa, Campylobacter spp, Campylobacter jejuni, Clostridium spp, Clostridium difficile, Mycobacterium spp, Mycobacterium tuberculosis, Treponema spp, Borrelia spp, Borrelia burgdorferi, Leptospira spp, Hemophilus ducreyi, Corynebacterium diphtheria, Bordetella pertussis, Bordetella parapertussis, Bordetella bronchiseptica, Hemophilus influenzae, Escherichia coli, Shigella spp, Ehrlichia spp et Rickettsia spp.

23. Procédé selon la revendication 22, dans lequel le polysaccharide est obtenu à partir d'un ou de plusieurs des éléments suivants :

   a. Streptococcus pneumoniae sérotype 1, 2, 3, 4, 5, 6, 6A, 6B, 6C, 6D, 6E, 6G, 6H, 7A, 7B, 7C, 7F, 8, 9A, 9L, 9F, 9N, 9V, 10F, 10B, 10C, 10A, 11A, 11F, 11B, 11C, 11D, 11E, 12A, 12B, 12F, 13, 14, 15A, 15C, 15B, 15F, 16A, 16F, 17A, 17F, 18, 18C, 18F, 18A, 18B, 19A, 19B, 19C, 19F, 20, 20A, 20B, 21, 22A, 22F, 23A, 23B, 23F, 24A, 24B, 24F, 25F, 25A, 27, 28F, 28A, 29, 31, 32F, 32A, 33A, 33C, 33D, 33E, 33F, 33B, 34, 45, 38, 35A, 35B, 35C, 35F, 36, 37, 38, 39, 40, 41F, 41A, 42, 43, 44, 45, 46, 47F, 47A, 48 ;
   b. Neisseria meningitidis sérotypes A, B, C, D, W135, X, Y, Z et 29E ;
   c. Haemophilus influenzae de type b ;
   d. Salmonella spp., y compris salmonella typhi, salmonella paratyphi A, salmonella paratyphi B, salmonella paratyphi C, salmonella typhimurium et salmonella enteritidis ;
   e. Streptococcus spp., y compris les streptocoques du groupe A et les streptocoques du groupe B, le groupe des streptocoques (Ia, Ib, II, III, IV, V, VI, VII, VIII et IX).

24. Procédé selon la revendication 1, dans lequel le vaccin conjugué polysaccharide-protéine à évaluer comprend une protéine porteuse choisie dans le groupe CRM197, toxine/anatoxine diphtérique, complexe de la membrane externe de Neisseria meningitidis, fragment C de l'anatoxine tétanique, toxine/anatoxine tétanique, toxine/anatoxine coquelucheuse, flagelline (FliC), sous-unité B de la toxine cholérique (CTB), protéine D de H. influenzae, E. coli LT, E. coli ST, et l'exotoxine A de Pseudomonas aeruginosa, le complexe de la membrane externe c (OMPC), les porines (Por A, Por B), les protéines de liaison à la transferrine, la pneumolysine, la protéine de surface du pneumocoque A (PspA), l'adhésine de surface du pneumocoque A (PsaA), PhtA, PhtB, PhtE, PhtD du pneumocoque, les protéines de surface du pneumocoque BVH-3 et BVH-11, l'uspA de M. catarrhalis, antigène protecteur (PA) de Bacillus anthracis et facteur d'œdème (EF) et facteur létal (LF) détoxifiés de Bacillus anthracis, ovalbumine, hémocyanine de patelle (KLH), C5a peptidase des streptocoques du groupe A ou du groupe B, sérum-albumine humaine, sérum-albumine bovine (BSA), protéine de poids moléculaire élevé de NTHi, fHbp, dérivé protéique purifié de la tuberculine (PPD) et sous-unité B de la toxine cholérique.

25. Procédé selon la revendication 23, dans lequel le polysaccharide est choisi parmi les sérotypes A, C, Y, W135 et X de Neisseria meningitidis.

26. Procédé selon la revendication 24, dans lequel la protéine porteuse est choisie parmi le CRM197, l'anatoxine tétanique (TT) et leurs combinaisons.

27. Procédé selon la revendication 1, dans lequel le vaccin conjugué polysaccharide-protéine est un vaccin multivalent.

28. Procédé selon la revendication 1, dans lequel le vaccin conjugué polysaccharide-protéine est un vaccin monovalent.

29. Procédé selon la revendication 27, dans lequel le vaccin multivalent comprend le conjugué TT du sérotype A de Neisseria meningitidis, le conjugué TT du sérotype X de Neisseria meningitidis, le conjugué CRM197 du sérotype C de Neisseria meningitidis, le conjugué CRM197 du sérotype Y de Neisseria meningitidis et le conjugué CRM197 du sérotype W de Neisseria meningitidis.

Fig. 1

Fig. 2

EP 4 145 124 B1

Fig. 3

Fig. 4

Fig. 5

1 Dose: Comparision betweeen Lyophilzed Vs Liquid Vaccine

■ 40°C (Lyo)   ▥ 40°C (Liq)

Fig. 6

1 Dose: Comparision betweeen Lyophilzed Vs Liquid Vaccine

■ 60°C (Lyo)   ▥ 60°C (Liq)

Fig. 7

5 Dose: Comparision betweeen Lyophilzed Vs Liquid Vaccine

■ 2-8°C (Lyo)   ■ 2-8°C (Liq)

Fig. 8

5 Dose: Comparision betweeen Lyophilzed Vs Liquid Vaccine

■ 25°C (Lyo)   ■ 25°C (Liq)

Fig. 9

5 Dose: Comparision betweeen Lyophilzed Vs Liquid Vaccine

Fig. 10

5 Dose: Comparision betweeen Lyophilzed Vs Liquid Vaccine

Fig. 11

2349T001 Lyo (2-8°C_1M)[2349T001_1 Month_27062022] Total peak processed
2349T001 Lyo (25°C_1M)[2349T001_1 Month_27062022] Total peak processed
2349T001 Lyo (40°C_1M)[2349T001_1 Month_27062022] Total peak processed
2349T001 Lyo (60°C_1M)[2349T001_1 Month_27062022] Total peak processed

(Green: 2-8°C, Brown: 25°C, Red: 40°C, Blue: 60°)

Fig. 12

2349T001 Lyo (2-8°C_1M)[2349T001_1 Month_27062022] Total peak processed

2349T001 Lyo (25°C_1M)[2349T001_1 Month_27062022] Total peak processed

2349T001 Lyo (40°C_1M)[2349T001_1 Month_27062022] Total peak processed

2349T001 Lyo (60°C_1M)[2349T001_1 Month_27062022] Total peak processed

(Green: 2-8°C, Brown: 25°C, Red: 40°C, Blue: 60°)

Fig. 13

2349T001 Lyo (2-8°C_1M)[2349T001_1 Month_27062022] Total peak processed

2349T001 Lyo (25°C_1M)[2349T001_1 Month_27062022] Total peak processed

2349T001 Lyo (40°C_1M)[2349T001_1 Month_27062022] Total peak processed

2349T001 Lyo (60°C_1M)[2349T001_1 Month_27062022] Total peak processed

(Green: 2-8°C, Brown: 25°C, Red: 40°C, Blue: 60°)

Fig. 14

2349T001 Liq (2-8°C_1M)[2349T001_1 Month_27062022] Total peak processed

2349T001 Liq (25°C_1M)[2349T001_1 Month_27062022] Total peak processed

2349T001 Liq (40°C_1M)[2349T001_1 Month_27062022] Total peak processed

2349T001 Liq (60°C_1M)[2349T001_1 Month_27062022] Total peak processed

(Green: 2-8°C, Brown: 25°C, Blue: 40°C, Red: 60°)

Fig. 15

2349T001 Liq (2-8°C_1M) [2349T001_1 Month_27062022] Total peak processed

2349T001 Liq (25°C_1M) [2349T001_1 Month_27062022] Total peak processed

2349T001 Liq (40°C_1M) [2349T001_1 Month_27062022] Total peak processed

2349T001 Liq (60°C_1M) [2349T001_1 Month_27062022] Total peak processed

(Green: 2-8°C, Brown: 25°C, Blue: 40°C, Red: 60°)

Fig. 16

2349T001 Liq (2-8°C_1M) [2349T001_1 Month_27062022] Total peak processed

2349T001 Liq (25°C_1M) [2349T001_1 Month_27062022] Total peak processed

2349T001 Liq (40°C_1M) [2349T001_1 Month_27062022] Total peak processed

2349T001 Liq (60°C_1M) [2349T001_1 Month_27062022] Total peak processed

(Green: 2-8°C, Brown: 25°C, Blue: 40°C, Red: 60°)

Fig. 17

2359T001 Lyo (2-8°C_1M)[2359T001_1 Month_23062022] Total peak processed

2359T001 Lyo (25°C_1M)[2359T001_1 Month_23062022] Total peak processed

2359T001 Lyo (40°C_1M)[2359T001_1 Month_23062022] Total peak processed

2359T001 Lyo (60°C_1M)[2359T001_1 Month_23062022] Total peak processed

(Brown: 2-8°C, Blue: 25°C, Red: 40°C, Green: 60°)

Fig. 18

2359T001 Lyo (2-8°C_1M)[2359T001_1 Month_23062022] Total peak processed
2359T001 Lyo (25°C_1M)[2359T001_1 Month_23062022] Total peak processed
2359T001 Lyo (40°C_1M)[2359T001_1 Month_23062022] Total peak processed
2359T001 Lyo (60°C_1M)[2359T001_1 Month_23062022] Total peak processed

(Brown: 2-8°C, Blue: 25°C, Red: 40°C, Green: 60°)

Fig. 19

2359T001 Liq (2-8°C_1M)[2359T001_1 Month_23062022] Total
peak processed
2359T001 Liq (25°C_1M)[2359T001_1 Month_23062022] Total peak
processed
2359T001 Liq (40°C_1M)[2359T001_1 Month_23062022] Total peak
processed
2359T001 Liq (60°C_1M)[2359T001_1 Month_23062022] Total peak
processed

(Brown: 2-8°C, Blue: 25°C, Red: 40°C, Green: 60°)

Fig. 20

2359T001 Liq (2-8°C_1M)[2359T001_1 Month_23062022] Total peak processed

2359T001 Liq (25°C_1M)[2359T001_1 Month_23062022] Total peak processed

2359T001 Liq (40°C_1M)[2359T001_1 Month_23062022] Total peak processed

2359T001 Liq (60°C_1M)[2359T001_1 Month_23062022] Total peak processed

(Green: 2-8°C, Brown: 25°C, Blue: 40°C, Red: 60°)

43

Fig. 21

▬▬ 2359T001 Liq (2-8°C_1M)[2359T001_1 Month_23062022] Total peak processed

▬▬ 2359T001 Liq (25°C_1M)[2359T001_1 Month_23062022] Total peak processed

▬▬ 2359T001 Liq (40°C_1M)[2359T001_1 Month_23062022] Total peak processed

▬▬ 2359T001 Liq (60°C_1M)[2359T001_1 Month_23062022] Total peak processed

(Green: 2-8°C, Brown: 25°C, Blue: 40°C, Red: 60°)

Fig. 22

2359T001 Liq (2-8°C_1M)[2359T001_1 Month_23062022] Total peak processed

2359T001 Liq (25°C_1M)[2359T001_1 Month_23062022] Total peak processed

2359T001 Liq (40°C_1M)[2359T001_1 Month_23062022] Total peak processed

2359T001 Liq (60°C_1M)[2359T001_1 Month_23062022] Total peak processed

(Green: 2-8°C, Brown: 25°C, Blue: 40°C, Red: 60°)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROSE-MARIE OLANDERA et al.** Booster response to the tetanus and diphtheria toxoid carriers of 11-valent pneumococcal conjugate vaccine in adults and toddlers. *Vaccine*, 2002, vol. 20, 336-341